# EUROPEAN PATENT APPLICATION

(11) **EP 4 357 364 A1**
(43) Date of publication of application: **24.04.2024**
(21) Application number: 22914112.2
(22) Date of filing: 07.12.2022
(51) Int. Cl.: C07K 16/28, G01N 33/574, C12N 15/13, A61K 39/395, A61K 47/68, A61P 35/00, A61P 37/02, A61P 37/06, A61P 31/00

(54) **ANTIBODY AND USE THEREOF**

(30) Priority: 28.12.2021 CN 202111629693
(71) Applicant: Hefei TG Immunopharma Co., Ltd., Hefei, Anhui 230001 (CN)
(72) Inventor: TIAN, Zhigang, Hefei, Anhui 230001 (CN); CAO, Guoshuai, Hefei, Anhui 230001 (CN); XIAO, Weihua, Hefei, Anhui 230001 (CN); SUN, Rui, Hefei, Anhui 230001 (CN); SUN, Haoyu, Hefei, Anhui 230001 (CN)
(74) Representative: Latscha Schöllhorn Partner AG
(86) International application number: PCT/CN2022/137233
(87) International publication number: WO 2023/124857

(57) **Abstract**

Disclosed is an antibody and use thereof. The antibody or antigen binding fragment includes CDR sequences selected from at least one of heavy-chain variable region CDR sequences: SEQ ID NOs: 1-3; and light-chain variable region CDR sequences: SEQ ID NOs: 4-6. The antibody prepared by the present disclosure can effectively bind to the α3 domain of MICA or MICB, and further, promote the binding of NKG2D to MICA and/or MICB, and can effectively treat or prevent related diseases mediated by MICA and/or MICB, such as promoting the killing of tumors by NK cell.

## Description

### PRIORITY INFORMATION

This application claims priority and the benefit of patent application No. 202111629693.7 submitted to the China National Intellectual Property Administration on December 28, 2021, which is incorporated herein by reference in its entirety.

### FIELD

The present disclosure belongs to the field of biopharmaceuticals, in particular to an antibody and use thereof, more particularly to an antibody or an antigen binding fragment, an immunoconjugate, a composition, a kit configured to detect MICA and/or MICB, use of the antibody or the antigen binding fragment in preparing a kit, a medicament, use of the antibody or the antigen binding fragment and/or the immunoconjugate and/or the composition for the preparation of a medicament, a nucleic acid, a recombinant vector or a transformant.

### BACKGROUND

NKG2D forms homodimers in NK cells and hexamers with four DAP10 molecules. After binding to MICA, MICB, and other ligands, NKG2D hexamers transmit activation signals to NK cells, promote NK cells to secrete cytokines such as IFN-γ, and kill target cells expressing ligands. MICA and MICB are proteins encoded by autologous genes, which are not expressed in normal tissues and cells; MICA and MICB express on the surface of tumor cells after malignant transformation. NK cells recognize and kill tumors through the NKG2D-MICA/B interaction.

Tumors can down-regulate MICA/B expression on the cell surface through various mechanisms, including the shedding of MICA/B mediated by matrix metalloproteinase. MICA/B is a type I transmembrane protein whose extracellular segment includes three domains, α1, α2, and α3, which bind to NKG2D through α1 and α2 domains. Under the action of matrix metalloproteinases, most of the amino acids of the extracellular segment of MICA/B (including the α1, α2 domains and part of the α3 domain) are shed from the tumor cell surface. NKG2D cannot bind to the part of the α3 domain that remains on the cell surface, so the tumor can escape immune surveillance of NK cells.

Studies have shown that α3 domain-binding MICA antibodies can inhibit the shedding of MICA from the tumor surface, thereby promoting NKG2D-MICA/B-mediated NK cell recognition and inhibiting tumor immune escape. Therefore, it is of great value to develop α3 domain-binding MICA antibody for tumor therapy.

### SUMMARY

The present disclosure is based on the discovery and recognition by the inventors of the following facts and problems:

Since MICA, MICB are expressed only in tumor cells, the preparation of specific antibodies targeting MICA, MICB is a potential means for treating tumors. After immunizing mice with MICA extracellular region Fc fusion protein (MICA-Fc) as an antigen, the inventors obtained mice containing anti-human MICA and MICB immunoglobulins through multiple screening and proceeded with subclone screening to obtain the target antibody, which can bind to α3 domain of MICA and/or MICB through detection, and further, promote the binding of MICA and/or MICB to NK cell activating receptor NKG2D, and promote the killing of tumors by NK cells.

Therefore, in a first aspect of the present disclosure, the present disclosure provides an antibody or antigen binding fragment. According to an embodiment of the present disclosure, including CDR sequences selected from at least one of heavy-chain variable region CDR sequences: SEQ ID NOs: 1-3; and light-chain variable region CDR sequences: SEQ ID NOs: 4-6. The antibody or antigen binding fragment according to an embodiment of the present disclosure can effectively bind to the α3 domain of MICA and/or MICB, and further, promote NKG2D binding to MICA and/or MICB, with a good effect in preventing and/or treating MICA and/or MICB-mediated diseases, such as promoting the killing of tumors by NK cells.
GFSLTTYG (SEQ ID NO: 1).
IWTDGTT (SEQ ID NO: 2).
VRKGHGYYYAMDY (SEQ ID NO: 3).
SSVSSSY (SEQ ID NO: 4).
STS (SEQ ID NO: 5).
HQYHRSPFT (SEQ ID NO: 6).

In a second aspect of the present disclosure, the present disclosure provides an immunoconjugate. According to an embodiment of the present disclosure, including the antibody or antigen binding fragment of the first aspect. As described above, the antibody or antigen binding fragment of an embodiment of the present disclosure can effectively bind to the α3 domain of the MICA and/or MICB protein, further, promoting the binding of NKG2D to MICA and/or MICB, with a good effect in preventing and/or treating MICA and/or MICB-mediated diseases, such as promoting the killing of tumors by NK cells.

In a third aspect of the present disclosure, the present disclosure provides a composition. According to an embodiment of the present disclosure, including the antibody as described in the first aspect and/or the immunoconjugate as described in the second aspect. As described above, the antibody or antigen binding fragment, and immunoconjugate of an embodiment of the present disclosure all can effectively bind to the α3 domain of MICA and/or MICB, and thus, the composition including the above substance has the same effect, and further, the composition can effectively promote NKG2D binding to MICA and/or MICB. The antibody or antigen binding fragment has a good effect in preventing and/or treating MICA and/or MICB-mediated diseases, such as promoting the killing of tumors by NK cells.

In a fourth aspect of the present disclosure, the present disclosure provides a kit configured to detect MICA and/or MICB. According to an embodiment of the present disclosure, including the antibody or antigen binding fragment of the first aspect. As described above, the antibody or antigen binding fragment of an embodiment of the present disclosure can effectively bind to the α3 domain of MICA and/or MICB, and thus, the antibody or antigen binding fragment can be used to detect MICA and/or MICB, and further, can be used to prepare a kit configured to detect MICA and/or MICB. The kit can effectively detect MICA and/or MICB and can be used in scientific research, such as qualitative or quantitative detection of MICA and/or MICB protein molecules in a biological sample, so as to obtain a biological sample that meets the requirements for further research. The biological sample includes animal tissues, blood, serum, cells and the like.

In a fifth aspect of the present disclosure, the present disclosure provides use of the antibody or antigen binding fragment of the first aspect in preparing a kit. According to an embodiment of the present disclosure, the kit is used for detecting MICA and/or MICB. As described above, the antibody or antigen binding fragment of an embodiment of the present disclosure can effectively bind to the α3 domain of MICA and/or MICB, and thus, the antibody or antigen binding fragment can be used to detect MICA and/or MICB, and further, can be used to prepare a kit configured to detect MICA and/or MICB.

In a six aspect of the present disclosure, the present disclosure provides a medicament, according to an embodiment of the present disclosure, including the antibody or antigen binding fragment of the first aspect and/or the immunoconjugate of the second aspect and/or the composition of the third aspect. As described above, the antibody or antigen binding fragment, immunoconjugate, and the composition of an embodiment of the present disclosure all have a substance that can effectively bind to the α3 domain of MICA and/or MICB, such that a medicament including the above substance can effectively bind to the α3 domain of MICA and/or MICB, and further, promote the binding of NKG2D to MICA and/or MICB, and further promote the action of NK cells, such as killing tumor cells. The medicament has remarkable effect in preventing and/or treating MICA and/or MICB-mediated diseases.

In a seventh aspect of the present disclosure, the present disclosure provides use of the antibody or antigen binding fragment of the first aspect and/or the immunoconjugate of the second aspect and/or the composition of the third aspect in the preparation of a medicament. According to an embodiment of the present disclosure, the medicament is used in preventing and/or treating of MICA and/or MICB-mediated diseases. As described above, the antibody or antigen binding fragment, the immunoconjugate, and the composition of an embodiment of the present disclosure all have a substance that can effectively bind to the α3 domain of MICA and/or MICB, such that a medicament including the above substance can effectively bind to the α3 domain of MICA and/or MICB, and further, promote the binding of NKG2D to MICA and/or MICB, and further promote the action of NK cells, such as killing tumor cells. The medicament has remarkable effect in preventing and/or treating MICA and/or MICB-mediated diseases.

In an eighth aspect of the present disclosure, the present disclosure provides a nucleic acid. According to an embodiment of the present disclosure, the nucleic acid encodes the antibody or antigen binding fragment of the first aspect. The antibody or antigen binding fragment encoded by the nucleic acid according to an embodiment of the present disclosure can effectively bind to the α3 domain of MICA and/or MICB, and further, promoting the binding of NKG2D to MICA and/or MICB. The protein encoded by the nucleic acid has a good effect in preventing and/or treating MICA and/or MICB-mediated diseases.

In a ninth aspect of the present disclosure, the present disclosure provides an expression vector or transformant. According to an embodiment of the present disclosure, including the nucleic acid of the eighth aspect. The expression vector may include optional control sequences operably linked to the nucleic acid molecule. Wherein the control sequences are one or more control sequences that can direct the expression of the nucleic acid molecule in a host. The recombinant vectors proposed by an embodiment of the present disclosure can highly express the antibody or antigen binding fragment in suitable host cells. The antibody or antigen binding fragment can effectively bind to the α3 domain of MICA and/or MICB. The antibody or antigen binding fragment has a good effect in preventing and/or treating MICA and/or MICB-mediated diseases, such as promoting the killing of tumors by NK cells.

In a tenth aspect of the present disclosure, the present disclosure provides a recombinant cell. According to an embodiment of the present disclosure, the recombinant cell carries the nucleic acid of the eighth aspect, the recombinant vector or transformant of the ninth aspect, or expresses the antibody or antigen binding fragment of the first aspect. The recombinant cell is obtained by transfecting or transforming the expression vector. According to an embodiment of the present disclosure, the recombinant cell can highly express the above-mentioned antibody under suitable conditions. The antibody can effectively bind to the α3 domain of MICA and/or MICB, and further, the antibody or antigen binding fragment has a good effect in preventing and/or treating MICA and/or MICB-mediated diseases, such as promoting the killing of tumors by NK cells.

In an eleventh aspect the present disclosure, the present disclosure provides use of the antibody or antigen binding fragment thereof, the immunoconjugate, the composition, the medicament, the nucleic acid, the recombinant vector or the transformant or the recombinant cell as described above in the treating and/or preventing of a MICA and/or MICB-mediated disease. As described above, the antibody or antigen binding fragment thereof can effectively bind to the α3 domain of MICA and/or MICB, further, promoting the binding of NKG2D to MICA and/or MICB, and further promoting the action of NK cells, such as killing tumor cells. Therefore, the antibody or antigen binding fragment thereof, and the substance capable of expressing the antibody or antigen binding fragment thereof under appropriate conditions can effectively prevent and/or treat MICA and/or MICB-mediated diseases.

In a twelfth aspect of the present disclosure, the present disclosure provides a method for treating and/or preventing a MICA and/or MICB-mediated disease. According to an embodiment of the present disclosure, the method includes administering to the subject at least one of 1) the antibody or antigen binding fragment thereof as described above; 2) the immunoconjugate as described above; 3) the composition as described above 4) the medicament as described above; 5) the nucleic acid as described above; 6) the recombinant vector or transformant as described above; and 7) the recombinant cell as described above. As described above, the antibody or antigen binding fragment thereof can effectively bind to the α3 domain of MICA and/or MICB, further, promoting the binding of NKG2D to MICA and/or MICB, and further promoting the action of NK cells, such as killing tumor cells. Therefore, the antibody or antigen binding fragment thereof, the substance capable of expressing the antibody or antigen binding fragment thereof under suitable conditions, or the substance comprising the antibody or antigen binding fragment thereof, can effectively prevent and/or treat MICA and/or MICB-mediated diseases. The method according to an embodiment of the present disclosure can effectively prevent and/or treat MICA and/or MICB-mediated diseases.

In a thirteenth aspect of the present disclosure, the present disclosure provides a method for diagnosing whether a subject has a MICA and/or MICB-mediated disease. According to an embodiment of the present disclosure, the method includes detecting MICA and/or MICB in a sample to be tested using at least one of the following: 1) the antibody or antigen binding fragment thereof as described above; 2) the nucleic acid as described above; 3) the recombinant vector or transformant as described above; and 4) the recombinant cell as described above; and determining the content of MICA and/or MICB in the sample to be test based on the detection result of the MICA and/or MICB. As described above, the antibody or antigen binding fragment of an embodiment of the present disclosure can effectively bind to the α3 domain of MICA and/or MICB, and therefore, the antibody or antigen binding fragment can be used to detect MICA and/or MICB, and MICA and/or MICB mediate a variety of diseases, and therefore, whether a subject has a MICA and/or MICB-mediated disease can be determined based on the level of the MICA and/or MICB contained in a biological sample from the subject. In addition, the above-mentioned substances can also be used for monitoring the content of MICA and/or MICB in the sample to be tested of a subject, namely, the above-mentioned substances can also be used for disease staging of a subject suffering from a MICA and/or MICB-mediated disease, and can also be used for evaluating the prognosis of a MICA and/or MICB-mediated disease.

In a fourteenth aspect of the present disclosure, the present disclosure provides use of at least one of the following in diagnosing whether a subject has a MICA and/or MICB-mediated disease: 1) the antibody or antigen binding fragment thereof as described above; 2) the nucleic acid as described above; 3) the recombinant vector or transformant as described above; and 4) the recombinant cell as described above. As described above, the antibody or antigen binding fragment of an embodiment of the present disclosure can effectively bind to the α3 domain of MICA and/or MICB, and therefore, the antibody or antigen binding fragment can be used to detect MICA and/or MICB, and MICA and/or MICB mediate a variety of diseases, and therefore, whether a subject has a MICA and/or MICB-mediated disease can be determined based on the level of the MICA and/or MICB contained in a biological sample from the subject.

Additional aspects and advantages of the present disclosure will be set forth in part in the description which follows and, in part, will be apparent from the following description, or may be learned by practice of the present disclosure.

### BRIEF DESCRIPTION OF DRAWINGS

The foregoing and/or additional aspects and advantages of the present disclosure will become apparent and readily appreciated from the following description of the embodiments, taken in conjunction with the accompanying drawings of which:
FIG. 1 is a graph of ELISA showing the binding of MICA antibody to human MICA according to a specific embodiment of the present disclosure;
FIG.2 is a graph of ELISA showing the binding of the MICA antibody to the α3 domain of MICA according to a specific embodiment of the present disclosure;
FIG. 3 is a graph showing the binding of the MICA antibody to CHO-K1-MICA according to a specific embodiment of the present disclosure;
FIG. 4 is a graph showing the binding of the MICA antibody to CHO-K1-MICB according to a specific embodiment of the present disclosure;
FIG.5 is a graph showing the binding of the MICA antibody to CHO-K1-Rhesus MIC1 according to a specific embodiment of the present disclosure;
FIG. 6 is a graph showing the binding of the MICA antibody to CHO-K1-Rhesus MICB according to a specific embodiment of the present disclosure;
FIG.7 is a graph showing the binding of the MICA antibody to melanoma A375 cells according to a specific embodiment of the present disclosure;
FIG. 8 is a graph showing the binding of the MICA antibody to lung cancer NCI-H1299 cells according to a specific embodiment of the present disclosure;
FIG.9 is a graph showing the binding of the MICA antibody to K562 cells according to a specific embodiment of the present disclosure;
FIG. 10 is a graph showing the Western blot detection of the MICA antibody according to a specific embodiment of the present disclosure;
FIG. 11 is a graph showing the effect of the MICA antibody on promoting the binding of NKG2D-Fc to CHO-K1-MICA cells according to a specific embodiment of the present disclosure;
FIG. 12 is a graph showing the effect of the MICA antibody on promoting the killing of A375 cells by NK92 cells according to a specific embodiment of the present disclosure;
FIG. 13 is a graph showing the binding of the MICA antibody to the MICAα3*002 variant according to a specific embodiment of the present disclosure;
FIG. 14 is a graph showing the binding of the MICA antibody to the MICAα3*004 variant according to a specific embodiment of the present disclosure;
FIG.15 is a graph showing the binding of the MICA antibody to the MICAα3*005 variant according to a specific embodiment of the present disclosure;
FIG. 16 is a graph showing the binding of the MICA antibody to the MICAα3*008 variant according to a specific embodiment of the present disclosure; and
FIG.17 is a graph showing the binding of the MICA antibody to the MICBα3*005 variant according to a specific embodiment of the present disclosure.

### DESCRIPTION OF EMBODIMENTS

Reference will now be made in detail to the embodiments of the present disclosure, examples of which are illustrated in the accompanying drawings. The embodiments described below with reference to the figures are exemplary and are intended to be illustrative of the present disclosure and are not to be construed as limiting the present disclosure.

Furthermore, the terms "first" and "second" are used for descriptive purposes only and are not to be construed as indicating or implying relative importance or implicitly indicating the number of technical features indicated. Thus, a feature defined with "first" or "second" may explicitly or implicitly comprises at least one feature. In the description of the present disclosure, the meaning of "plurality" is at least two, e.g. two, three, etc. unless specifically limited otherwise.

The endpoints of the ranges and any values disclosed herein are not limited to the precise range or value, and such ranges or values should be understood to include values approximating such ranges or values. For numerical ranges, the endpoints of each range, the endpoints of each range and the individual point values, and the individual point values, can be combined with each other to produce one or more new numerical ranges, and such numerical ranges are to be considered to be specifically disclosed herein.

For an easier understanding of the present disclosure, certain technical and scientific terms are specifically defined below. Unless clearly defined elsewhere in this document, all other technical and scientific terms used herein have the meanings commonly understood by general technical personnel in the field to which the present disclosure belongs. Abbreviations for amino acid residues are standard 3-letter and/or 1-letter codes used in the art to refer to one of the 20 commonly used L-amino acids.

Herein, certain regions in the variable region have a higher degree of variation in amino acid composition and arrangement, referred to as Hypervariable region (HVR), which is the location where antigen and antibody bind, and thus also referred to as complementarity-determining region (CDR). There are three CDR regions on both the heavy-chain variable region and light-chain variable region.

As used herein, an "antibody" as previously described, an antibody of the present disclosure may be full-length (e.g. IgG1 or IgG4 antibody) or include only an antigen binding portion (e.g. Fab, F(ab') 2, or scFv fragment), or may be modified to affect function. The present disclosure includes anti-MICA antibodies having modified glycosylation patterns. In some applications, it may be useful to make modifications to remove undesired glycosylation sites, or to eliminate the presence of fucose moieties on the oligosaccharide chains, e.g. to enhance antibody-dependent ADCC function. In other applications, galactosylation modifications can be made to alter complement dependent cytotoxicity (CDC).

The term "antigen binding fragment" as used herein refers in particular to a partial fragment of an antibody, such as Fv, scFv (sc means single chain), Fab, F(ab')2, Fab', scFv-Fc fragment or diabody, or any fragment which should be capable of increasing the half-life by chemical modification, e.g. addition of polyalkylene glycols, such as polyethylene glycol ("PEGylation, PEG") (referred to as pegylated fragments of Fv-PEG, scFv-PEG, Fab-PEG, F(ab')2-PEG or Fab'-PEG) ("PEG" means polyethylene glycol) or by incorporation into liposomes, said fragments having MICA and/or MICB binding activity. Preferably, the antigen binding fragment will consist of partial sequences of the heavy-chain variable region or light-chain variable region of its source antibody, or contain partial sequences of light-chain variable region and heavy-chain variable region, sufficient to retain the same binding specificity and sufficient affinity as its source antibody. For and/or MICB, it is preferred to be at least 1/100 of its source antibody affinity, and in a more preferred manner, at least 1/10. Such antigen binding fragments will include a minimum of 5 amino acids, preferably 10, 15, 25, 50, and 100 contiguous amino acids of the antibody sequence from which they are derived.

Herein, in order to further improve the biological acceptability of the antibodies, the antibodies may also be humanized, i.e. the antibodies are chimeric or humanized. The term "chimeric antibody" refers to a recombinant antibody obtained by replacing the amino acid sequence of the constant region of a monoclonal antibody from one species (e.g. mouse) with the constant region of an antibody from another species (e.g. human) using recombinant DNA technology. The term "humanized antibody" refers to a recombinant antibody obtained by completely replacing the constant region and the non-CDR (Fv framework region (FR)) amino acid sequences of the variable region of a monoclonal antibody from one species (e.g. mouse) with the constant region and non-CDR amino acid sequences of the variable region of an antibody from another species (e.g. human) using recombinant DNA technology. That is when the constant region of an antibody is humanized, it is referred to as a chimeric antibody, and after all of the constant and the non-CDR amino acid sequences of the variable region are humanized, it is referred to as a humanized antibody. Methods of humanization may be carried out with reference to conventional antibody engineering techniques and will not be described in detail herein.

Homology, in the present disclosure, in order to compare two or more nucleotide sequences, the percentage of "sequence homology" between a first sequence and a second sequence may be calculated by dividing the number of nucleotides in the first sequence that are identical to the nucleotides at the corresponding positions. Nucleotides in the second sequence] minus [total number of nucleotides in the first sequence] and then multiplied by [100%], wherein the deletion, insertion, substitution, or addition of each nucleotide in the second nucleotide sequence relative to the first nucleotide sequence is considered to be a difference in a single nucleotide (position).

Alternatively, the degree of sequence identity between two or more nucleotide sequences can be calculated using standard settings by using known computer algorithms for sequence alignment, such as NCBI Blast v2.0.

Some other techniques, computer algorithms, and settings for determining the degree of sequence identity are for example described in WO 04/037999, EP 0 967 284, EP 1 085 089, WO 00/55318, WO 00/78972, WO 98/49185 and GB 2357768-A.

For polypeptides, the term "(substantial) homology" means that at least about 80%, usually at least about 90% to 95%, and more preferably at least about 98% to 99.5% of the amino acids of two polypeptides or designated sequences thereof are identical when optimally aligned and compared (with appropriate insertions or deletions of nucleotides).

The % homology between two sequences varies with the number of identical positions shared by the sequences when optimally aligning the sequences (i.e.% homology = number of identical positions/total number of positions × 100), where optimal alignment is determined taking into account the number of gaps that need to be introduced to achieve optimal alignment of the two sequences and the length of each gap. Sequence comparison and percent identity determination between two sequences can be accomplished using mathematical algorithms, as described in the non-limiting examples below.

One skilled in the art may substitute, add and/or delete one or more (e.g. 1, 2, 3, 4, 5, 6, 7, 8, 9, or 10 or more) amino acids to a sequence of the present disclosure to obtain a variant of the sequence of the antibody or functional fragment thereof without substantially affecting the activity of the antibody (retaining at least 95% activity). They are considered to be included within the scope of the present disclosure. For example, amino acids having similar properties in the variable region may be substituted. The sequence of the variant of the present disclosure may have at least 80%, 85%, 90%, 95%, 96%, 97%, 98%, or 99% identity (or homology) to the reference sequence. Sequence identity described herein can be measured using sequence analysis software, e.g. a computer program BLAST using default parameters, in particular BLASTP or TBLASTN. Amino acid sequences referred to herein are shown from the N-terminus to the C-terminus.

As used herein, a "conservative modification form of an amino acid sequence" refers to an amino acid modification that does not significantly affect or alter the binding characteristics of an antibody comprising the amino acid sequence, including amino acid substitutions, additions, and deletions. Modifications can be introduced into the antibody of the present disclosure by standard techniques such as site-directed mutagenesis and PCR-mediated mutagenesis. Conservative amino acid substitution refers to the substitution of amino acid residues by amino acid residues with similar side chains. Families of amino acid residues having similar side chains have been identified in the art. These families include amino acids with basic side chains, e.g. lysine, arginine, histidine, amino acids with acidic side chains, e.g. aspartic acid, glutamic acid, amino acids with uncharged polar side chains, e.g. glycine, asparagine, glutamine, serine, threonine, tyrosine, cysteine, tryptophan, amino acids with nonpolar side chains, e.g. alanine, valine, leucine, isoleucine, proline, phenylalanine, methionine, amino acids with β-branched side chains, e.g. threonine, valine, isoleucine, and amino acids with aromatic side chains, e.g. tyrosine, phenylalanine, tryptophan, histidine. Thus, one or more of the amino acid residues in the CDR region of an antibody of the present disclosure can be replaced with other amino acid residues from the same side chain family, and the altered antibody can be tested for retained function using the functional assays described herein. Preferably, the number of conservative modifications does not exceed one or two.

As used herein, "operably linked" means that the foreign gene is linked to a vector such that control elements within the vector, such as a transcription control sequence and a translation control sequence, etc. are capable of performing their intended function of regulating the transcription and translation of the foreign gene.

In one aspect of the present disclosure, the present disclosure provides an antibody or antigen binding fragment comprising a CDR sequence selected from at least one of: heavy-chain variable region CDR sequences: SEQ ID NOs: 1-3 or an amino acid sequence in a conservative modification form thereof; light-chain variable region CDR sequences: SEQ ID NO: 4-6 or an amino acid sequence in a conservative modification form thereof. The conservative modification of any one of amino acid sequences as set forth in SEQ ID NOs: 1-6 does not significantly affect or alter the binding characteristics of an antibody or antigen binding fragment comprising the amino acid sequence, and therefore an antibody or antigen binding fragment comprising at least one of SEQ ID NOs: 1-6 and/or at least one of an amino acid sequence obtained by conservative modification of an amino acid sequence as set forth in SEQ ID NOs: 1-6 according to an embodiment of the present disclosure can effectively bind to the α3 domain of MICA and/or MICB, and further, promote the binding of NKG2D to MICA and/or MICB, may be useful in the treating or preventing of related MICA and/or MICB-mediated diseases, such as promoting the killing of tumors by NK cells.
GFSLTTYG (SEQ ID NO: 1).
IWTDGTT (SEQ ID NO: 2).
VRKGHGYYYAMDY (SEQ ID NO: 3).
SSVSSSY (SEQ ID NO: 4).
STS (SEQ ID NO: 5).
HQYHRSPFT (SEQ ID NO: 6).

According to some specific embodiments of the present disclosure, the above-mentioned antibody or antigen binding fragment may further include at least one of the following additional technical features:
According to some specific embodiments of the present disclosure, included are a heavy chain CDR1 with an amino acid sequence as set forth in SEQ ID NO: 1 or conservative modification form thereof, a heavy chain CDR2 with an amino acid sequence as set forth in SEQ ID NO: 2 or conservative modification form thereof, a heavy chain CDR3 with an amino acid sequence as set forth in SEQ ID NO: 3 or conservative modification form thereof, a light chain CDR1 with an amino acid sequence as set forth in SEQ ID NO: 4 or conservative modification form thereof, a light chain CDR2 with an amino acid sequence as set forth in SEQ ID NO: 5 or conservative modification form thereof, and a light chain CDR3 with an amino acid sequence as set forth in SEQ ID NO: 6 or conservative modification form thereof.

According to some specific embodiments of the present disclosure, the gene encoding the heavy-chain variable region CDR has at least one of the nucleotide sequences as set forth in SEQ ID NOs: 15-17.
GGGTTTTCACTGACAACTTACGGC (SEQ ID NO: 15).
ATTTGGACTGATGGGACCACA (SEQ ID NO: 16).
GTGCGGAAGGGCCATGGGTACTACTATGCCATGGATTAT (SEQ ID NO: 17).

According to some specific embodiments of the present disclosure, the gene encoding the light-chain variable region CDR has at least one of the nucleotide sequences as set forth in SEQ ID NOs: 18-20.
AGCAGCGTGTCCAGCTCCTAC (SEQ ID NO: 18).
TCCACCTCC (SEQ ID NO: 19).
CATCAGTATCATAGGTCCCCTTTCACT (SEQ ID NO: 20).

According to some specific embodiments of the present disclosure, a heavy-chain variable region and a light-chain variable region are included, wherein (i) the heavy-chain variable region includes an amino acid sequence having at least 80% homologous to at least one of an amino acid sequence as set forth in SEQ ID NO: 7 and a conservative modification form thereof; and/or, (ii) the light-chain variable region includes an amino acid sequence having at least 80% homologous to at least one of an amino acid sequence as set forth in SEQ ID NO: 8 and a conservative modification form thereof.

QVQLKQSGPGLVQPSQSLSITCIVSGFSLTTYGVHWVRQSPGKGLEWLGMI
WTDGTTDYNAAFISRLSINKDNSKSQVFLKMNSLQADDTGIYYCVRKGHGYYYAMD
YWGQGTSVTVSS (SEQ ID NO: 7).

QILLTQSPAILSASLGERVTMTCTASSSVSSSYLHWYQQKPGSSPKLWIYSTS
NLASGVPARFSGGGSGTSYSLTVSNMEAEDAATYYCHQYHRSPFTFGSGTKLEIK
(SEQ ID NO: 8).

According to some specific embodiments of the present disclosure, the gene encoding the heavy-chain variable region has a nucleotide sequence as set forth in SEQ ID NO: 21.

CAGGTGCAGTTGAAACAGTCTGGTCCTGGGCTTGTACAGCCATCTCAGT
CTCTGTCTATTACCTGTATTGTGTCCGGGTTTTCACTGACAACTTACGGCGTGCACT
GGGTTAGGCAGTCTCCAGGTAAGGGCTTGGAGTGGCTTGGCATGATTTGGACTGAT
GGGACCACAGACTACAACGCTGCTTTCATCAGTCGCCTGTCCATCAATAAGGACAA
CTCTAAAAGCCAGGTATTTCTGAAGATGAACAGCTTGCAAGCTGACGACACCGGA
ATTTACTACTGCGTGCGGAAGGGCCATGGGTACTACTATGCCATGGATTATTGGGGT
CAGGGCACCAGCGTCACAGTATCCTCT (SEQ ID NO: 21).

According to some specific embodiments of the present disclosure, the gene encoding the light-chain variable region has a nucleotide sequence as set forth in SEQ ID NO: 22.

CAGATCCTGTTGACCCAAAGTCCAGCTATTCTTTCAGCCTCTTTGGGCG

AACGTGTGACTATGACCTGCACTGCTTCCAGCAGCGTGTCCAGCTCCTACTTGCAC
TGGTATCAGCAGAAGCCTGGTTCATCCCCTAAGCTGTGGATCTATTCCACCTCCAA
CCTCGCTTCAGGAGTGCCTGCCAGATTTTCCGGAGGCGGAAGTGGCACTTCCTACT
CACTTACAGTCAGCAACATGGAGGCAGAGGACGCAGCTACCTACTACTGCCATCA
GTATCATAGGTCCCCTTTCACTTTCGGGTCCGGTACCAAGCTCGAGATCAAA(SEQ
ID NO: 22).

According to some specific embodiments of the present disclosure, the heavy-chain variable region includes an amino acid sequence having at least 90%, at least 95%, at least 96%, at least 97%, at least 98%, at least 99%, or 100% homologous to the heavy-chain variable region selected from (i); the light-chain variable region includes an amino acid sequence having at least 90%, at least 95%, at least 96%, at least 97%, at least 98%, at least 99%, or 100% homologous to the light-chain variable region selected from (ii).

According to some specific embodiments of the present disclosure, further included is an Fc region, at least a portion of which is derived from at least one of a murine antibody, a human antibody, a primate antibody, or a mutant thereof

According to some specific embodiments of the present disclosure, at least a portion of the Fc region is derived from a murine, a human, a primate IgG antibody, or a mutant thereof.

According to some specific embodiments of the present disclosure, at least a portion of the Fc region is derived from a murine IgG 2a antibody, a human, a primate IgG1 antibody, or a mutant thereof.

According to some specific embodiments of the present disclosure, at least a portion of the Fc region is derived from a murine IgG 2a antibody or a mutant thereof.

According to some specific embodiments of the present disclosure, the Fc region has an amino acid sequence as set forth in SEQ ID NO: 14.

AKTTAPSVYPLAPVCGDTTGSSVTLGCLVKGYFPEPVTLTWNSGSLSSGVH
TFPAVLQSDLYTLSSSVTVTSSTWPSQSITCNVAHPASSTKVDKKIEPRGPTIKPCPPCK
CPAPNLLGGPSVFIFPPKIKDVLMISLSPIVTCVVVDVSEDDPDVQISWFVNNVEVHTA
QTQTHREDYNSTLRVVSALPIQHQDWMSGKEFKCKVNNKDLPAPIERTISKPKGSVR
APQVYVLPPPEEEMTKKQVTLTCMVTDFMPEDIYVEWTNNGKTELNYKNTEPVLDS

DGSYFMYSKLRVEKKNWVERNSYSCSVVHEGLHNHHTTKSFSRTPGK(SEQ ID NO:
14).

According to some specific embodiments of the present disclosure, the antibody or antigen binding fragment includes a heavy chain comprising an amino acid sequence as set forth in SEQ ID NO: 9 and a light chain comprising an amino acid sequence as set forth in SEQ ID NO: 10.

QVQLKQSGPGLVQPSQSLSITCIVSGFSLTTYGVHWVRQSPGKGLEWLGMI
WTDGTTDYNAAFISRLSINKDNSKSQVFLKMNSLQADDTGIYYCVRKGHGYYYAMD
YWGQGTSVTVSSAKTTAPSVYPLAPVCGDTTGSSVTLGCLVKGYFPEPVTLTWNSGS
LSSGVHTFPAVLQSDLYTLSSSVTVTSSTWPSQSITCNVAHPASSTKVDKKIEPRGPTIK
PCPPCKCPAPNLLGGPSVFIFPPKIKDVLMISLSPIVTCVVVDVSEDDPDVQISWFVNN
VEVHTAQTQTHREDYNSTLRVVSALPIQHQDWMSGKEFKCKVNNKDLPAPIERTISKP
KGSVRAPQVYVLPPPEEEMTKKQVTLTCMVTDFMPEDIYVEWTNNGKTELNYKNTE
PVLDSDGSYFMYSKLRVEKKNWVERNSYSCSVVHEGLHNHHTTKSFSRTPGK (SEQ
ID NO: 9).

QILLTQSPAILSASLGERVTMTCTASSSVSSSYLHWYQQKPGSSPKLWIYSTS
NLASGVPARFSGGGSGTSYSLTVSNMEAEDAATYYCHQYHRSPFTFGSGTKLEIKRAD
AAPTVSIFPPSSEQLTSGGASVVCFLNNFYPKDINVKWKIDGSERQNGVLNSWTDQDS
KDSTYSMSSTLTLTKDEYERHNSYTCEATHKTSTSPIVKSFNRNEC (SEQ ID NO: 10).

According to some specific embodiments of the present disclosure, wherein the antibody or antigen binding fragment is IgG1, IgG2, or IgG4.

According to some specific embodiments of the present disclosure, the antibody is a monoclonal antibody, a murine antibody, a chimeric antibody, a humanized antibody, a human antibody, Fv, a single chain antibody (scFv), Fab, Fab', Fab'-SH or F(ab')2.

According to some specific embodiments of the present disclosure, the antibody or antigen binding fragment thereof can bind to an amino acid sequence as set forth in SEQ ID NO: 11.

MGLGPVFLLLAGIFPFAPPGAAAEPHSLRYNLTVLSWDGSVQSGFLTEVHL
DGQPFLRCDRQKCRAKPQGQWAEDVLGNKTWDRETRDLTGNGKDLRMTLAHIKDQ
KEGLHSLQEIRVCEIHEDNSTRSSQHFYYDGELFLSQNLETKEWTMPQSSRAQTLAM

NVRNFLKEDAMKTKTHYHAMHADCLQELRRYLKSGVVLRRTVPPMVNVTRSEASE
GNITVTCRASGFYPWNITLSWRQDGVSLSHDTQQWGDVLPDGNGTYQTWVATRICQ
GEEQRFTCYMEHSGNHSTHPVPSGKVLVLQSHWQTFHVSAVAAAAIFVIIIFYVRCCK
KKTSAAEGPELVSLQVLDQHPVGTSDHRDATQLGFQPLMSDLGSTGSTEGA (SEQ ID
NO: 11).

In another aspect of the present disclosure, the present disclosure provides an immunoconjugate comprising a therapeutic agent and an antibody or antigen binding fragment as described above, coupled to the therapeutic agent. As described above, the antibody or antigen binding fragment of an embodiment of the present disclosure can effectively bind to the α3 domain of the MICA and/or MICB protein and, further, promoting the binding of NKG2D to MICA and/or MICB, with a good effect in preventing and/or treating MICA and/or MICB-mediated diseases, such as promoting the killing of tumors by NK cells. The immunoconjugate obtained by coupling the antibody or antigen binding fragment with the therapeutic agent also has the aforementioned functions.

In a further aspect, the present disclosure provides a composition comprising an antibody or antigen binding fragment thereof as described above and/or an immunoconjugate as described above. As described above, the antibody or antigen binding fragment, and immunoconjugate of an embodiment of the present disclosure all can effectively bind to the α3 domain of MICA and/or MICB, and thus, the composition comprising the same, such as a food composition or pharmaceutical composition, has the same effect, and further, the composition is effective to promote NKG2D binding to MICA and/or MICB, with a good effect in preventing and/or treating MICA and/or MICB-mediated diseases, such as promoting the killing of tumors by NK cells.

According to a specific embodiment of the present disclosure, the composition further includes a pharmaceutically acceptable carrier.

It should be noted that the composition includes combinations that are separated in time and/or space so long as they can work together to achieve the objects of the present disclosure. For example, the components contained in the composition can be administered to subjects as a whole or separately. When the components contained in the composition are administered separately to a subject, the individual components may be administered to the subject simultaneously or sequentially.

In one aspect, the present disclosure provides a kit configured to detect MICA and/or MICB, wherein the kit includes the antibody or antigen binding fragment as described above. As described above, the antibody or antigen binding fragment of an embodiment of the present disclosure can effectively bind to the α3 domain of MICA and/or MICB, and thus, the antibody or antigen binding fragment can be used to detect MICA and/or MICB, and further, can be used to prepare a kit configured to detect MICA and/or MICB. The kit can effectively detect MICA and/or MICB and can be used in scientific research, such as qualitative or quantitative detection of MICA and/or MICB protein molecules in a biological sample. More specifically, it can be used for immunoblotting, immunoprecipitation, and other kits related to the use of MICA and/or MICB proteins and their antibody-specific binding characteristics to detect. These kits may include any one or more of the following: antagonist, MICA, and/or MICB antibody or drug reference material; a protein purification column; an immunoglobulin affinity purification buffer; an assay diluent for cells. MICA and/or MICB antibodies can be used for different types of diagnostic tests, e.g. in vitro or in vivo to detect the presence of a wide variety of diseases or drugs, toxins or other proteins, etc. For example, MICA and/or MICB-related diseases can be tested by testing the subject's serum or blood.

In a further aspect, the present disclosure provides use of the antibody or antigen binding fragment thereof as described above in preparing a kit configured to detect MICA and/or MICB. As described above, the antibody or antigen binding fragment of an embodiment of the present disclosure can effectively bind to the α3 domain of MICA and/or MICB. Therefore, the antibody can be used for detecting MICA and/or MICB, and further, the antibody can be used in preparing a kit configured to detect MICA and/or MICB. The kit can efficiently detect MICA and/or MICB and can be used for scientific studies, such as qualitative or quantitative detection of MICA and/or MICB protein molecules in biological samples, more particularly, can be used for immunoblotting, immunoprecipitation, and other kits that involve the use of the specific binding performance of MICA and/or MICB and its antibody or binding fragment to detect. These kits may include any one or more of the following: antagonist, MICA, and/or MICB antibody or drug reference material; a protein purification column; an immunoglobulin affinity purification buffer; an assay diluent for cells. MICA and/or MICB antibodies or antigenic fragments can be used for different types of diagnostic tests, e.g. in vitro or in vivo to detect a wide variety of diseases or the presence of drugs, toxins or other proteins, etc. For example, MICA and/or MICB-related diseases can be tested by testing the subject's serum or blood.

In yet another aspect of the present disclosure, the present disclosure provides a medicament comprising an antibody or antigen binding fragment as described above and/or a conjugate as described above and/or a composition as described above for use in preventing and/or treating a MICA and/or MICB mediated disease. As described above, the antibody or antigen binding fragment, immunoconjugate, and composition of an embodiment of the present disclosure all have a substance that can effectively bind to the α3 domain of MICA and/or MICB. Therefore, a medicament comprising the above substances can effectively bind to the α3 domain of MICA and/or MICB, and further, promote the binding of NKG2D to MICA and/or MICB, which has a remarkable effect in preventing and/or treating MICA and/or MICB-mediated diseases.

According to some specific embodiments of the present disclosure, the above-mentioned medicament may further include at least one of the following additional technical features:
According to some embodiments of the present disclosure, the MICA and/or MICB-mediated disease is a cancer, a transplant rejection, an autoimmune disease, or an infectious disease.

According to some specific embodiments of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

According to a specific embodiment of the present disclosure, further included are a pharmaceutically acceptable carrier and an effective amount of the antibody active ingredient.

As used herein, the term "effective amount" or "effective dose" refers to an amount that produces a function or activity and is accepted by humans and/or animals.

As used herein, a "pharmaceutically acceptable" ingredient is one that is suitable for use in humans and/or mammals without undue adverse side effects (such as toxicity, irritation, and allergic response), i.e. one that has a reasonable benefit/risk ratio. The term "pharmaceutically acceptable carrier" refers to a carrier used in the administration of therapeutic agents, including various excipients and diluents.

The medicament of the present disclosure contains a safe and effective amount of the active ingredient of the present disclosure and a pharmaceutically acceptable carrier. Such carriers include but are not limited to saline, buffer, glucose, water, glycerol, ethanol, and combinations thereof. Generally, the pharmaceutical preparation should be matched with the administration mode, and the dosage form of the medicament of the present disclosure can be an injection, an oral preparation (tablet, capsule, and oral liquid), a transdermal agent, or a sustained-release agent. They are prepared, for example, by conventional methods using physiological saline or aqueous solutions containing glucose and other adjuvants. The medicament is preferably manufactured under sterile conditions.

The effective amount of the active ingredient described in the present disclosure can vary depending on the mode of administration and the severity of the disease to be treated. The selection of the optimal effective amount can be determined by ordinary technicians in the field based on various factors (such as through clinical trials). Such factors include, but are not limited to pharmacokinetic parameters of the active ingredient such as bioavailability, metabolism, half-life, and the like; the severity of the disease of the patient to be treated, the weight of the patient, the immune status of the patient, the route of administration, etc. For example, several divided doses may be administered daily or the dose may be proportionally reduced as indicated by the exigencies of the therapeutic situation.

Pharmaceutically acceptable carriers described herein include but are not limited to water, saline, liposomes, lipids, proteins, protein-antibody conjugates, peptides, cellulose, nanogel, or combinations thereof. The selection of carriers should be matched with the drug delivery method, which is well-known to ordinary technical personnel in this field.

In one aspect of the present disclosure, the present disclosure provides a nucleic acid comprising a sequence encoding an antibody or antigen binding fragment thereof as described above. The antibody or antigen binding fragment encoded by the nucleic acid according to an embodiment of the present disclosure can effectively bind to the α3 domain of MICA and/or MICB, and further, promoting the binding of NKG2D to MICA and/or MICB. The protein encoded by nucleic acid has a good effect in preventing and/or treating MICA and/or MICB-mediated diseases.

According to a specific embodiment of the present disclosure, the nucleic acid has a nucleotide sequence as set forth in SEQ ID NOs: 12 and 13.

The gene encoding the heavy chain of the antibody or antigen binding fragment has the nucleotide sequence shown below:

CAGGTGCAGTTGAAACAGTCTGGTCCTGGGCTTGTACAGCCATCTCAGT
CTCTGTCTATTACCTGTATTGTGTCCGGGTTTTCACTGACAACTTACGGCGTGCACT
GGGTTAGGCAGTCTCCAGGTAAGGGCTTGGAGTGGCTTGGCATGATTTGGACTGAT
GGGACCACAGACTACAACGCTGCTTTCATCAGTCGCCTGTCCATCAATAAGGACAA
CTCTAAAAGCCAGGTATTTCTGAAGATGAACAGCTTGCAAGCTGACGACACCGGA
ATTTACTACTGCGTGCGGAAGGGCCATGGGTACTACTATGCCATGGATTATTGGGGT
CAGGGCACCAGCGTCACAGTATCCTCTGCCAAAACAACTGCACCATCTGTTTATCC
ACTGGCCCCCGTTTGCGGGGATACCACTGGTAGCTCTGTCACCCTCGGCTGTCTGG
TCAAAGGATATTTCCCCGAGCCTGTGACACTTACCTGGAATTCAGGCAGCTTGTCC
TCCGGCGTGCATACTTTCCCTGCAGTCCTGCAGTCAGATCTGTACACCCTCAGCTC
ATCTGTGACTGTCACAAGTTCTACCTGGCCATCCCAGAGTATTACATGCAACGTGG
CCCACCCAGCAAGTTCAACAAAAGTTGACAAGAAGATTGAGCCAAGAGGCCCTA
CTATCAAGCCCTGTCCCCCCTGTAAGTGTCCTGCACCCAATCTGCTGGGGGGCCCA
TCTGTGTTTATTTTCCCCCCAAAGATTAAGGACGTCCTCATGATTAGCCTGTCCCCA
ATCGTGACATGTGTGGTGGTGGATGTTTCTGAGGACGACCCAGACGTACAGATCTC
TTGGTTCGTGAACAATGTCGAGGTGCATACAGCCCAGACCCAGACCCATCGGGAA
GACTACAACTCTACATTGAGAGTGGTGTCCGCTTTGCCCATCCAGCATCAGGACTG
GATGTCCGGCAAGGAGTTTAAATGTAAGGTCAACAACAAGGACCTGCCCGCTCCA
ATAGAGAGAACTATCTCAAAGCCTAAAGGTAGTGTTCGAGCCCCCCAGGTATACGT
ACTGCCACCCCCTGAGGAGGAGATGACCAAGAAGCAGGTGACACTGACCTGCAT
GGTGACAGACTTCATGCCAGAAGACATTTATGTCGAATGGACTAATAATGGCAAGA
CCGAACTGAATTATAAAAATACCGAACCAGTGCTGGACTCCGACGGGTCCTATTTC
ATGTACTCTAAGCTCCGTGTCGAAAAGAAGAACTGGGTGGAACGAAACTCTTACT

CCTGCAGTGTTGTGCACGAGGGGCTTCACAACCATCATACAACCAAGTCCTTCTCC
AGGACACCTGGGAAG (SEQ ID NO: 12).

The gene encoding the light chain of the antibody or antigen binding fragment has the nucleotide sequence shown below:

CAGATCCTGTTGACCCAAAGTCCAGCTATTCTTTCAGCCTCTTTGGGCG
AACGTGTGACTATGACCTGCACTGCTTCCAGCAGCGTGTCCAGCTCCTACTTGCAC
TGGTATCAGCAGAAGCCTGGTTCATCCCCTAAGCTGTGGATCTATTCCACCTCCAA
CCTCGCTTCAGGAGTGCCTGCCAGATTTTCCGGAGGCGGAAGTGGCACTTCCTACT
CACTTACAGTCAGCAACATGGAGGCAGAGGACGCAGCTACCTACTACTGCCATCA
GTATCATAGGTCCCCTTTCACTTTCGGGTCCGGTACCAAGCTCGAGATCAAACGAG
CTGATGCTGCCCCCACTGTCTCTATTTTTCCTCCTAGTAGTGAGCAGCTGACATCTG
GAGGCGCCTCCGTGGTGTGCTTCCTGAATAACTTTTACCCTAAGGACATCAATGTG
AAGTGGAAGATCGACGGGAGTGAGAGGCAGAATGGCGTACTGAATTCCTGGACTG
ATCAGGACTCTAAAGATAGCACCTATAGCATGTCCTCCACCCTCACACTGACAAAG
GACGAATACGAACGGCATAACTCCTATACATGTGAGGCTACCCACAAGACATCCAC
CTCACCAATCGTCAAGAGCTTCAACCGGAATGAGTGT (SEQ ID NO: 13).

It should be noted that for the nucleic acid mentioned in the specification and claims of the present disclosure, those skilled in the art should understand that it actually includes either or both complementary double strands. For convenience, in this description and claims, while in most cases only one strand is shown, in fact, another strand complementary thereto is disclosed. In addition, the nucleic acid sequences of the present disclosure include DNA forms or RNA forms, one of which is disclosed, meaning that the other is also disclosed.

In another aspect of the present disclosure, the present disclosure provides a recombinant vector or transformant containing the nucleic acid described above. The recombinant vector may include optional control sequences operably linked to the nucleic acid molecule. Wherein the control sequences are one or more control sequences that can direct the expression of the nucleic acid molecule in a host. The recombinant vectors proposed by an embodiment of the present disclosure can highly express the antibody or antigen binding fragment in suitable host cells. The antibody or antigen binding fragment can effectively bind to the α3 domain of MICA and/or MICB. The antibody or antigen binding fragment has a good effect in preventing and/or treating MICA and/or MICB-mediated diseases, such as promoting the killing of tumors by NK cells.

The recombinant vector may refer to a cloning vector or an expression vector and may be obtained by operably linking the nucleic acid with a commercially available vector such as a plasmid or a viral vector. The recombinant vector of the present disclosure is not particularly limited, and commonly used plasmids such as pSeTag2, PEE14, pMH3, etc. may be used.

In a further aspect of the present disclosure, the present disclosure provides a recombinant cell carrying a nucleic acid, recombinant vector, or transformant as described above or expressing the antibody or antigen binding fragment as described above. The recombinant cell is obtained by transfecting or transforming the expression vector. According to an embodiment of the present disclosure, the recombinant cell can highly express the above-mentioned antibody under suitable conditions. The antibody can effectively bind to the α3 domain of MICA and/or MICB, and further, the antibody or antigen binding fragment has a good effect in preventing and/or treating MICA and/or MICB-mediated diseases, such as promoting the killing of tumors by NK cells.

It is to be noted that the recombinant cell of the present disclosure is not particularly limited and may be a prokaryotic cell, a eukaryotic cell or a bacteriophage. The prokaryotic cell may be *E. coli*, *Bacillus subtilis*, *Streptomyces* or *Proteus mirabilis*, etc. The eukaryotic cells may be fungi such as *Pichia pastoris*, *Saccharomyces cerevisiae*, *Schizosaccharomyces pombe*, *Trichoderma*, insect cells such as *Mythimna separata*, plant cells such as tobacco, mammalian cells such as BHK cells, CHO cells, COS cells, myeloma cells, and the like. In some embodiments, the recombinant cells of the present disclosure are preferably mammalian cells, including BHK cells, CHO cells, NSO cells, or COS cells, and do not include animal germ cells, fertilized eggs, or embryonic stem cells.

It should be noted that "suitable conditions" in the description of the present disclosure refers to the conditions suitable for the expression of the recombinant antibodies described in the present disclosure. Those skilled in the art will readily appreciate that suitable conditions for recombinant antibody expression include, but are not limited to, suitable transformation or transfection manners, suitable transformation or transfection conditions, healthy host cell status, suitable host cell density, suitable cell culture environment, and suitable cell culture time. The "suitable conditions" are not particularly limited, and those skilled in the art can optimize the most suitable conditions for the expression of the recombinant antibody according to the specific environment of the laboratory.

In one aspect the present disclosure, the present disclosure provides the use of the antibody or antigen binding fragment thereof, immunoconjugate, composition, medicament, nucleic acid, recombinant vector or transformant or recombinant cell as described above in treating and/or preventing of a MICA and/or MICB-mediated disease. As described above, the antibody or antigen binding fragment thereof can effectively bind to the α3 domain of MICA and/or MICB, further, promoting the binding of NKG2D to MICA and/or MICB, and further promoting the action of NK cells, such as killing tumor cells. Therefore, the antibody or antigen binding fragment thereof, and the substance capable of expressing the antibody or antigen binding fragment thereof under appropriate conditions can effectively prevent and/or treat MICA and/or MICB-mediated diseases.

According to a specific embodiment of the present disclosure, the above-mentioned use in treating and/or preventing of a MICA and/or MICB-mediated disease may further include at least one of the following additional technical features:
According to a specific embodiment of the present disclosure, the MICA and/or MICB-mediated disease is a cancer, a transplant rejection, an autoimmune disease, or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

In another aspect of the present disclosure, the present disclosure provides a method for treating and/or preventing MICA and/or MICB-mediated diseases. According to a specific embodiment of the present disclosure, the method includes administering to the subject at least one of 1) the antibody or antigen binding fragment thereof as described above 2) the immunoconjugate as described above; 3) the composition as described above 4) the medicament as described above; 5) the nucleic acid as described above; 6) the recombinant vector or transformant as described above; and 7) the recombinant cell as described above. As described above, the antibody or antigen binding fragment thereof can effectively bind to the α3 domain of MICA and/or MICB, further, promoting the binding of NKG2D to MICA and/or MICB, and further promoting the action of NK cells, such as killing tumor cells. Therefore, the antibody or antigen binding fragment thereof, the substance capable of expressing the antibody or antigen binding fragment thereof under suitable conditions, or the substance including the antibody or antigen binding fragment thereof, can effectively prevent and/or treat MICA and/or MICB-mediated diseases. The method according to an embodiment of the present disclosure can effectively prevent and/or treat MICA and/or MICB-mediated diseases.

According to a specific embodiment of the present disclosure, the above method may further include at least one of the following additional technical features:
According to a specific embodiment of the present disclosure, the MICA and/or MICB-mediated disease is a cancer, a transplant rejection, an autoimmune disease, or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

In another aspect of the present disclosure, the present disclosure provides a method for diagnosing whether a subject has MICA and/or MICB-mediated diseases. According to a specific embodiment of the present disclosure, the method includes detecting the MICA and/or MICB in a sample to be tested using at least one of the following: 1) the antibody or antigen binding fragment thereof as described above; 2) the nucleic acid as described above; 3) the recombinant vector or transformant as described above; and 4) the recombinant cell as described above; and determining the content of MICA and/or MICB in the sample to be tested based on the detection results of the MICA and/or MICB. As described above, the antibody or antigen binding fragment of an embodiment of the present disclosure can effectively bind to the α3 domain of MICA and/or MICB, and therefore, the antibody or antigen binding fragment can be used to detect MICA and/or MICB, and MICA and/or MICB mediate a variety of diseases, and therefore, whether a subject has MICA and/or MICB-mediated diseases can be determined based on the level of the MICA and/or MICB contained in a biological sample from the subject. In addition, the above-mentioned substances can also be used for monitoring the content of MICA and/or MICB in the sample to be tested of a subject, namely, the above-mentioned substances can also be used for disease staging of a subject suffering from a MICA and/or MICB-mediated disease, and can also be used for prognosis of MICA and/or MICB-mediated disease.

According to a specific embodiment of the present disclosure, the above method may further include at least one of the following additional technical features:
According to a specific embodiment of the present disclosure, a content of MICA and/or MICB in the sample to be tested that is not lower than the minimum standard for disease is an indication that the sample to be tested is derived from a patient having a MICA and/or MICB-mediated disease. The value of the minimum standard can be determined by comparing, analyzing and verifying the difference in the content of MICA and/or MICB in the sample to be tested from a large number of individuals with the MICA and/or MICB-mediated disease and a large number of healthy individuals.

According to a specific embodiment of the present disclosure, the MICA and/or MICB-mediated disease is a cancer, a transplant rejection, an autoimmune disease, or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

According to a specific embodiment of the present disclosure, the sample to be tested includes at least one of the following: tissue, cells, blood, serum, sweat, feces, and urine.

In another aspect of the present disclosure, the present disclosure provides use of at least one of following for diagnosing whether a subject has a MICA and/or MICB-mediated disease: 1) the antibody or antigen binding fragment thereof as described above; 2) the nucleic acid as described above; 3) the recombinant vector or transformant as described above; and 4) the recombinant cell as described above. As described above, the antibody or antigen binding fragment of an embodiment of the present disclosure can effectively bind to the α3 domain of MICA and/or MICB, and therefore, the antibody or antigen binding fragment can be used to detect MICA and/or MICB, and MICA and/or MICB mediate a variety of diseases, and therefore, whether a subject has MICA and/or MICB-mediated diseases can be determined based on the level of the MICA and/or MICB contained in a biological sample from the subject.

According to a specific embodiment of the present disclosure, the above uses may further include at least one of the following additional technical features:
According to a specific embodiment of the present disclosure, the MICA and/or MICB-mediated disease is a cancer, a transplant rejection, an autoimmune disease, or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

Hereinafter, examples will be described in detail. Where specific techniques or conditions are not specified in the examples, they are performed according to techniques or conditions described in the literature in the art or according to the product specification. The reagents or instruments used without specifying the manufacturer are all conventional products that can be obtained commercially

### Examples 1 Preparation of antibody

This example mainly includes the following contents.

Balb/c mice (9-week-old, purchased from Shanghai SLAC, weighing about 20 g) were immunized with purified recombinant MICA extracellular region Fc fusion protein (MICA-Fc) (recombinant MICA extracellular region Fc fusion protein, amino acid sequence as set forth in SEQ ID NO: 23) as antigen to generate murine monoclonal antibody against human MICA. The immunized mice were immunized 3 times with purified antigen and complete Freund's adjuvant. After the immunized mice were bled by the tail vein, they were detected by ELISA and flow cytometry to obtain the mice with anti-human MICA immunoglobulin.

EPHSLRYNLTVLSWDGSVQSGFLTEVHLDGQPFLRCDRQKCRAKPQGQWA
EDVLGNKTWDRETRDLTGNGKDLRMTLAHIKDQKEGLHSLQEIRVCEIHEDNSTRSS
QHFYYDGELFLSQNLETKEWTMPQSSRAQTLAMNVRNFLKEDAMKTKTHYHAMHA
DCLQELRRYLKSGVVLRRTVPPMVNVTRSEASEGNITVTCRASGFYPWNITLSWRQD
GVSLSHDTQQWGDVLPDGNGTYQTWVATRICQGEEQRFTCYMEHSGNHSTHPVPSG
KVLVLQSHWPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNH
YTQKSLSLSPGK (SEQ ID NO: 23).

A mouse with the highest content of anti-MICA immunoglobulin was chosen from the obtained mice containing anti-human MICA immunoglobulin, and the spleen cells thereof were taken out and fused with mouse myeloma cell SP2/0 cells (ATCC No. CRL-1581) to prepare a hybridoma. The specific experimental operation was a routine operation in this field. The fused hybridoma cells were used for antibody screening to obtain murine monoclonal antibodies.

The candidate hybridoma cells were cultured to a total number of 10⁶. The cells were collected by centrifugation at 800 rpm for 10 min, and the total RNA of hybridoma cells was extracted with Trizol kit (Invitrogen). The total RNA was used as a template to synthesize cDNA library (Invitrogen) by reverse transcription, and cDNA was used as a template to amplify the nucleic acid sequence of the variable region corresponding to the antibody contained in hybridoma cells by PCR. The primer sequences used in the PCR amplification reaction were complementary to the first framework region of the antibody variable region or signal peptide region and the constant region (Larrick, J.W., et al., (1990) Scand_J.Immunol., 32, 121-128 and Coloma, J.J.et al., (1991) BioTechniques, 11, 152-156). In a 50 µL reaction system, 2 µL of cDNA, 5 µL of 10×PCR buffer, 2 µL of upstream and downstream primers (5 µmol), 2 µL of dNTP, 1 µL of Taq enzyme (Takara, Ex Taq), and 38 µL of H₂O were added respectively. A pre-denaturation was performed for 5 min at 95°C, and the procedure was entered into the temperature cycle for PCR amplification. The reaction conditions included: denaturation at 94°C for 30 s, annealing at 58°C for 45 s, extension at 72°C for 50 S for 32 cycles, then extension at 72°C for 7 min. After sequencing the amplification products, the nucleotide sequences of the heavy-chain variable (SEQ ID NO: 21) and the light-chain variable (SEQ ID NO: 22) of the murine monoclonal antibody were obtained. Amino acid sequences of the heavy-chain variable (SEQ ID NO: 7) and the light-chain variable (SEQ ID NO: 8) of the murine monoclonal antibody were obtained.

CAGGTGCAGTTGAAACAGTCTGGTCCTGGGCTTGTACAGCCATCTCAGT
CTCTGTCTATTACCTGTATTGTGTCCGGGTTTTCACTGACAACTTACGGCGTGCACT
GGGTTAGGCAGTCTCCAGGTAAGGGCTTGGAGTGGCTTGGCATGATTTGGACTGAT
GGGACCACAGACTACAACGCTGCTTTCATCAGTCGCCTGTCCATCAATAAGGACAA
CTCTAAAAGCCAGGTATTTCTGAAGATGAACAGCTTGCAAGCTGACGACACCGGA
ATTTACTACTGCGTGCGGAAGGGCCATGGGTACTACTATGCCATGGATTATTGGGGT
CAGGGCACCAGCGTCACAGTATCCTCT (SEQ ID NO: 21).

CAGATCCTGTTGACCCAAAGTCCAGCTATTCTTTCAGCCTCTTTGGGCG
AACGTGTGACTATGACCTGCACTGCTTCCAGCAGCGTGTCCAGCTCCTACTTGCAC
TGGTATCAGCAGAAGCCTGGTTCATCCCCTAAGCTGTGGATCTATTCCACCTCCAA
CCTCGCTTCAGGAGTGCCTGCCAGATTTTCCGGAGGCGGAAGTGGCACTTCCTACT
CACTTACAGTCAGCAACATGGAGGCAGAGGACGCAGCTACCTACTACTGCCATCA
GTATCATAGGTCCCCTTTCACTTTCGGGTCCGGTACCAAGCTCGAGATCAAA (SEQ
ID NO: 22).

QVQLKQSGPGLVQPSQSLSITCIVSGFSLTTYGVHWVRQSPGKGLEWLGMI
WTDGTTDYNAAFISRLSINKDNSKSQVFLKMNSLQADDTGIYYCVRKGHGYYYAMD
YWGQGTSVTVSS (SEQ ID NO: 7).

QILLTQSPAILSASLGERVTMTCTASSSVSSSYLHWYQQKPGSSPKLWIYSTS
NLASGVPARFSGGGSGTSYSLTVSNMEAEDAATYYCHQYHRSPFTFGSGTKLEIK
(SEQ ID NO: 8).

### Example 2 ELISA binding assay of MICA antibody

The ELISA assay was used to detect the binding characteristics of the MICA antibody obtained in Example 1. The MICA extracellular region Fc fusion protein (MICA-Fc) as described above was coated into a 96-well plate. The signal strength after antibody addition was used to determine the binding characteristics of the antibody to MICA.

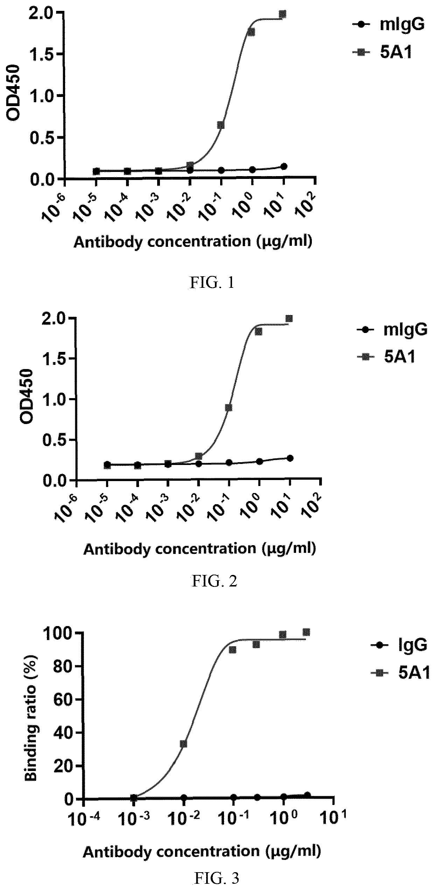

The MICA-Fc fusion protein (amino acid sequence as set forth in SEQ ID NO: 23) was diluted to 1 µg/mL with PBS buffer and added to a 96-well plate in a volume of 100 µL/well and left overnight at 4°C. The PBS buffer solution was sucked off from the 96-well plate. The plate was washed 6 times with PBST (pH 7.2, PBS containing 0.1% Tween 20) buffer solution. 200 µL/well PBS/10% BSA was added and incubated at 37°C for 2 hours for blocking. The blocking solution was removed. The plate was washed 6 times with PBST. The MICA antibody to be tested was diluted to an appropriate concentration with 100 µL/well PBST/0.05% BSA, and incubated for 1 h at 37°C. The reaction system was removed. The plate was washed 6 times with PBST. HRP (horseradish peroxidase)-labeled anti-mouse antibody secondary antibody (Boster, BA1050) was diluted with PBST/0.05% BSA at 100 µL/well and incubated for 1h at 37°C. After the incubation, the plate was washed 6 times with PBST, and 80 µL/well TMB (tetramethylbenzidine) was added. After 3 min of incubation at room temperature, and 80 µL/well 4M sulfuric acid was added to terminate the reaction. The absorbance at 450 mm was read with a microplate reader. The specific experimental results are shown in FIG. 1, indicating that the antibodies of the present disclosure can bind to MICA.

EPHSLRYNLTVLSWDGSVQSGFLTEVHLDGQPFLRCDRQKCRAKPQGQWA
EDVLGNKTWDRETRDLTGNGKDLRMTLAHIKDQKEGLHSLQEIRVCEIHEDNSTRSS
QHFYYDGELFLSQNLETKEWTMPQSSRAQTLAMNVRNFLKEDAMKTKTHYHAMHA
DCLQELRRYLKSGVVLRRTVPPMVNVTRSEASEGNITVTCRASGFYPWNITLSWRQD
GVSLSHDTQQWGDVLPDGNGTYQTWVATRICQGEEQRFTCYMEHSGNHSTHPVPSG
KVLVLQSHWPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNH
YTQKSLSLSPGK (SEQ ID NO: 23).

### Example 3 ELISA assay for binding of MICA antibody

The ELISA assay was used to detect the binding characteristics of the MICA antibodies. The MICA extracellular domain α3 domain Fc fusion protein (MICA α3-Fc) was coated into a 96-well plate. The signal strength after antibody addition was used to determine the binding characteristics of the antibody and MICA.

The MICAα3-Fc fusion protein (amino acid sequence as set forth in SEQ ID NO: 24) was diluted to 1 µg/mL with PBS buffer and added to a 96-well plate in a volume of 100 µL/well and left overnight at 4°C. The PBS buffer solution was sucked off from the 96-well plate. The plate was washed 6 times with PBST (pH 7.2, PBS containing 0.1% Tween 20) buffer solution. 200 µL/well PBS/10% BSA was added and incubated at 37°C for 2 hours for blocking. The blocking solution was removed. The plate was washed 6 times with PBST. The MICA antibodies to be tested were diluted to an appropriate concentration with 100 µL/well PBST/0.05% BSA, and incubated at 37°C for 1 h. The reaction system was removed. The plate was washed 6 times with PBST. HRP (horseradish peroxidase)-labeled anti-mouse antibody secondary antibody (Boster, BA1050) was diluted with PBST/0.05% BSA at 100 µL/well and incubated for 1 h at 37°C. The plate was washed 6 times with PBST, and 80 µL/well TMB (tetramethylbenzidine) was added. After 3 min of incubation at room temperature, and 80 µL/well 4M sulfuric acid was added to terminate the reaction. The absorbance at 450 mm was read with a microplate reader. The results (FIG.2) show that the antibody of the present disclosure can bind to the MICA α3 domain.

VLRRTVPPMVNVTRSEASEGNITVTCRASGFYPWNITLSWRQDGVSLSHD
TQQWGDVLPDGNGTYQTWVATRICQGEEQRFTCYMEHSGNHSTHPVPSGKVLVLQS
HWPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNK
ALPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLS
LSPGK (SEQ ID NO: 24).

### Example 4 Construction of CHO-K1 overexpression cell

HEK293T cells were plated in a six-well plate at 5×10⁵ cells/well and cultured overnight in a DMEM medium without diabody. The medium was discarded before transfection and 1 mL of fresh DMEM medium without diabody was added. The pLUX-EF1a-IRES-puro vector containing the target gene (nucleotide sequences encoding MICA (SEQ ID NO: 25), MICB (SEQ ID NO: 26), Rhesus MIC1 (SEQ ID NO: 27), and Rhesus MICB (SEQ ID NO: 28) (Synthesized by Suzhou GENEWIZ) were inserted between the restriction endonuclease sites EcoRI and BamHI of the pLVX-EF1a-IRES Pruo-vector, respectively), pMD2G, and psPAX2 vectors (a total of 3 µg) were added in 200 µL of serum-free DMEM medium in a 2:1:1 ratio, and 12 µg of polyether imide (PEI, Polysciences Co., Ltd.) was added. After mixing, the mixture was stood for 16 min. Then the whole liquid was added to a six-well plate plated with HEK293T cells. After 6 h of culture, the medium was discarded and a fresh complete DMEM medium was added for culture. 48 h after transfection, the cell culture supernatant was collected and passed through a 0.45 µm filter (Millipore) to obtain the virus supernatant. All viral supernatant was added to 6-well plates containing 1×10⁴ CHO-K1 cells, the polybrene (Sigma) at a final concentration of 4 µg/mL was added, and incubated for 12 h. The supernatant was then discarded and fresh complete DMEM medium was added. The resulting cells were CHO-K1-MICA, CHO-K1-MICB, CHO-K1-Rhesus MIC1, and CHO-K1-Rhesus MICB cells, which can express MICA (SEQ ID NO: 11), MICB (SEQ ID NO: 29), Rhesus MIC1 (SEQ ID NO: 30) and Rhesus MICB (SEQ ID NO: 31) proteins, respectively.

ATGGGCCTTGGCCCTGTGTTTCTGTTGCTTGCTGGGATTTTTCCATTCGC
ACCTCCCGGTGCCGCCGCAGAGCCCCACAGCTTGCGGTACAATTTGACCGTGCTG
TCCTGGGATGGCAGTGTACAAAGCGGCTTTCTGACCGAGGTTCATCTGGATGGAC
AACCATTCCTGCGCTGCGACAGACAGAAGTGTCGAGCAAAACCCCAGGGTCAGT
GGGCAGAAGATGTACTCGGCAATAAGACATGGGATCGAGAAACAAGAGACCTCAC
AGGAAATGGTAAAGATCTCCGCATGACCCTTGCACACATTAAGGATCAGAAAGAA
GGACTGCACTCCCTCCAGGAGATTCGAGTGTGTGAGATTCACGAGGATAACAGCA
CAAGAAGCTCACAGCATTTTTATTACGATGGAGAACTGTTTCTCAGCCAAAACCTG
GAGACCAAAGAGTGGACTATGCCACAGTCCTCCAGAGCTCAGACCCTGGCTATGA
ATGTCCGTAATTTTTTGAAAGAGGACGCAATGAAGACCAAGACTCACTACCATGCC
ATGCACGCCGACTGCCTGCAGGAGTTGAGACGATACCTCAAATCCGGGGTTGTGC
TCAGGCGGACAGTGCCCCCAATGGTAAACGTGACTCGGTCTGAAGCTTCTGAAGG
AAACATCACAGTCACCTGCAGAGCTAGCGGTTTTTACCCCTGGAACATCACTCTCT
CCTGGAGACAGGACGGTGTGTCCCTCAGCCATGATACCCAGCAGTGGGGAGACGT
GCTGCCCGATGGCAACGGCACTTATCAGACTTGGGTGGCTACTCGAATTTGTCAAG

GAGAGGAGCAGCGATTCACATGTTACATGGAGCACTCCGGGAATCATTCAACTCA
CCCAGTACCCAGCGGAAAGGTCCTTGTGCTGCAGAGTCATTGGCAGACATTTCAC
GTATCCGCTGTGGCAGCCGCCGCAATTTTCGTCATCATCATTTTCTATGTTCGCTGTT
GTAAAAAAAAGACAAGCGCTGCCGAGGGGCCCGAACTGGTGAGCCTCCAGGTGC
TGGACCAACATCCCGTGGGCACCAGCGACCATCGCGATGCCACCCAGCTTGGCTT
CCAGCCATTGATGTCAGACCTCGGCAGCACAGGCAGCACTGAGGGCGCT (SEQ ID
NO: 25).

ATGGGATTGGGTAGGGTTTTGCTTTTTCTCGCTGTGGCTTTCCCCTTTGC
ACCTCCTGCTGCAGCCGCAGAACCCCATAGCCTTAGGTATAACCTGATGGTGCTCA
GTCAAGATGAATCCGTGCAGTCCGGATTTCTTGCCGAGGGCCATCTGGATGGACAA
CCTTTCTTGCGTTACGATAGGCAGAAACGACGAGCTAAACCACAGGGTCAGTGGG
CAGAGGACGTGCTGGGAGCTAAGACTTGGGACACCGAGACCGAGGATCTGACTG
AGAACGGGCAGGACCTGAGAAGGACTCTGACACACATCAAGGACCAGAAGGGAG
GGTTGCACTCCCTGCAGGAAATCCGCGTGTGTGAGATTCACGAGGACTCATCAAC
CCGAGGCAGTAGGCACTTTTACTACGATGGAGAGCTGTTTCTGAGTCAGAATCTGG
AGACACAAGAGAGCACTGTGCCACAGTCATCTCGGGCTCAGACTCTGGCTATGAA
CGTTACCAATTTCTGGAAAGAAGATGCTATGAAAACAAAGACCCATTATAGAGCTA
TGCAGGCCGATTGCCTGCAGAAGTTGCAGCGTTATTTGAAGTCTGGTGTTGCTATC
AGGCGGACCGTTCCCCCCATGGTGAATGTGACTTGCTCCGAGGTTTCCGAAGGTA
ACATTACTGTTACCTGTCGAGCTAGCTCCTTCTACCCACGGAACATTACATTGACAT
GGAGGCAGGATGGCGTTAGTCTGAGCCATAATACTCAGCAGTGGGGAGACGTTCT
GCCTGACGGAAATGGCACATACCAGACCTGGGTTGCCACCAGAATCCGACAGGGC
GAAGAGCAGAGGTTTACCTGTTATATGGAGCACAGCGGAAATCATGGGACTCATCC
CGTGCCTAGTGGGAAGGTGCTCGTTCTCCAGTCTCAGCGCACTGACTTTCCTTACG
TGTCTGCCGCTATGCCATGCTTCGTCATCATTATCATCCTCTGTGTGCCATGCTGCAA
GAAGAAAACATCTGCAGCCGAGGGGCCTGAACTTGTGTCCCTGCAGGTTCTGGAC
CAGCACCCTGTTGGCACCGGAGATCACCGAGACGCAGCCCAACTGGGCTTCCAAC
CTCTGATGAGCGCCACTGGATCTACTGGTTCTACCGAGGGAGCC (SEQ ID NO: 26).

ATGGGCCCTGGGAGGGTTTTGCTCTTTCTGACCTCTATTTTTCCCTTCGC
TAGACCAGGCGCTCCAACAGAGCTCCACAGCTTGCGCTACAACGTGACAGTTCTC

AGCAGGGATGGGTCCGTGCAGTCCGATTTCCTCGCTGAGGGCCATCTTGATAGCCA
ACTCTTCGTCCGCTACGACCGCGAGACACGGCGGGCAAGGCCACAAGGACAGTG
GGCCGAGGCTGTGCTGGGCGCAAAAACATGGGACACTGAAACTGGCGATCTCAC
AGAGAATGGGAAGGACCTGCGGCGTACCCTGACTCACATTGAGGGACAAAAGGG
TGGCCTCCACAGCTTGCAGGATATACAGAATTGTGAGATTTACGAGGACGGCTCTA
CTGGAGGTTCCAGGCATTTTTACTATGATGGGGAGAGATTTCTGTCCCTTAATCTGG
CCACTCAGGAGTGGACAGTAGCACAGTCTAGCCGAGCACAGACTCTGGCCATGAA
CTTCTGGAAGGAAGATACTATGAAAACAAATACACACTACCACGCAATGCAGGCC
GATTGCCTGAAAAAGCTGAGACGCTACCAAAAGTCCAGAGTGGCTGTACGACGCA
CCGCCCCTCCTATGGTGAATGTGACACATTCCGAGGCTAGTGAGGGTAATATTACA
GTGACCTGCCGCGCTTCTGGTTTCTACCCCAGAAATATAGCCTTGACCTGGCGACA
GGACGGTGTTTCATTGAACCACGACGCCCAGCAGTGGGGCGGCATACTGCCAGAC
CAGAATGGCACTTACCAGACTTGGGTGGCCACCAGGATTCGGCAGGGCGAGGAAC
AGCGTTTCGCCTGCTATATGGAGCATAGCGGTAATCACTCTACCCATCCCGTGCCTA
GCGGAAAGGTCCTGGTCTTTCAATCTCAGTGGCTGGATATTCCATACGTGCTGGGT
GTAGCCGCTGCCGCCGTGGCCGCCGCTGCCGCAATTTTCGTTATTATCCTGTACGTT
CTGTGTTGCAAGAAAAAGACTTCAGCCGCCGAAGGCCCCGAGCTCGTGAGTCTGC
GCACCCTGGATCAGCACCCCGTTGGTACTGGAGATCACCGTGACGCAACC (SEQ ID
NO: 27).

ATGGGTCTGGGAAGAGTATTGCTGTTTCTGGGTCGAGTCCTGCTGTTTCT
CGCCTCCATTTTCTCTTTTACTCGACCTCGAGCCGCTGCTGAGCTGCACTCACTCA
GATACAACGTGACCGTCTTGTCTAGAGATGGGTCAGTGCAGAGCGAGTTTTTGGC
AGAGGGCCATTTGGATGGTCAGCTCTTTGTGAGGTACGATAGGGAAACACGACGT
GCTAGACCTCAGGGACAGTGGGCAGAGGCAGTGTTGGGGGACCAGGAGACAGAG
GACTTGACCGAGAACGCACAGGACCTGCGCAGAACACTGACCCACATTGAAGGC
CAGAAGGGCGGCCTGCATAGTCTGCAGAAGATAAAAATCTGCGAAATTTATGAGG
ATGGCTCTACTGGCGGATTTTGGCATTTTTATTACGACGGGGAGCTGTTTCTGTCCC
TTAATCTGGAGACACAGAAGTGGACAGTAGCTCAGTCATCCCGCGCTCAAACCCT
GGCAATGAATTTCTGGAAGGAGGACACTATGAAAACTAATACACACTATAGAGCCA
TGAGGGCCGATTGCTTGAAAAAACTGTGGAGATACCAGAAGTCTCGGGTTGCTGT

GAGAAGGACAGTTCCCCCTATGGTGAATGTCACTCATGGGGAGGCCTCTGAGGGC
AACATAACAGTCACATGCAGGGCTTCAGGCTTTTACCCTGGAAACATCACTCTGAC
TTGGAGACAAGATGGCGTGTCCCTGAGTCACGACGCACAACAGTGGGGCGACGT
CCTGCCTGATTGGAATGGCACCTATCAGACATGGGTCGCCACAAGAATCCGACAG
GGAGAGGAACAGCGTTTTGCCTGTTACATGGAGCATAGCGGCAATCATTCCACACA
TCCTGTGCCTTCTGGCAAGCCACCAGTGCTCCAGTCCGAACGGTTGAATCTGCTGT
ATGTCCCCGTAGCAGTGGCTGTTGCTGTTGTCACTGCCTTTATCATTATCTGCGTTC
ACCGATGTAAAAAGAAAAAAACATCAGCTGCCGAGGGCCCAGAGTTGGTATCCCT
CAGAACTCTTGACCAACACCCTGTCGGAACTGGGGATCATAGAGATGCTACCCAG
CTGGGATTCCAGCCTCTCATGAGCGCTCCAGGGTCCACCGGATCAACTGAAGGGG
CC (SEQ ID NO: 28).

MGLGPVFLLLAGIFPFAPPGAAAEPHSLRYNLTVLSWDGSVQSGFLTEVHL
DGQPFLRCDRQKCRAKPQGQWAEDVLGNKTWDRETRDLTGNGKDLRMTLAHIKDQ
KEGLHSLQEIRVCEIHEDNSTRSSQHFYYDGELFLSQNLETKEWTMPQSSRAQTLAM
NVRNFLKEDAMKTKTHYHAMHADCLQELRRYLKSGVVLRRTVPPMVNVTRSEASE
GNITVTCRASGFYPWNITLSWRQDGVSLSHDTQQWGDVLPDGNGTYQTWVATRICQ
GEEQRFTCYMEHSGNHSTHPVPSGKVLVLQSHWQTFHVSAVAAAAIFVIIIFYVRCCK
KKTSAAEGPELVSLQVLDQHPVGTSDHRDATQLGFQPLMSDLGSTGSTEGA (SEQ ID
NO: 11).







 (SEQ ID NO:
29).

MGPGRVLLFLTSIFPFARPGAPTELHSLRYNVTVLSRDGSVQSDFLAEGHLD
SQLFVRYDRETRRARPQGQWAEAVLGAKTWDTETGDLTENGKDLRRTLTHIEGQKG
GLHSLQDIQNCEIYEDGSTGGSRHFYYDGERFLSLNLATQEWTVAQSSRAQTLAMNF

WKEDTMKTNTHYHAMQADCLKKLRRYQKSRVAVRRTAPPMVNVTHSEASEGNITVT
CRASGFYPRNIALTWRQDGVSLNHDAQQWGGILPDQNGTYQTWVATRIRQGEEQRF
ACYMEHSGNHSTHPVPSGKVLVFQSQWLDIPYVLGVAAAAVAAAAAIFVIILYVLCCK
KKTSAAEGPELVSLRTLDQHPVGTGDHRDAT (SEQ ID NO: 30).

MGLGRVLLFLGRVLLFLASIFSFTRPRAAAELHSLRYNVTVLSRDGSVQSEF
LAEGHLDGQLFVRYDRETRRARPQGQWAEAVLGDQETEDLTENAQDLRRTLTHIEGQ
KGGLHSLQKIKICEIYEDGSTGGFWHFYYDGELFLSLNLETQKWTVAQSSRAQTLAM
NFWKEDTMKTNTHYRAMRADCLKKLWRYQKSRVAVRRTVPPMVNVTHGEASEGNI
TVTCRASGFYPGNITLTWRQDGVSLSHDAQQWGDVLPDWNGTYQTWVATRIRQGEE
QRFACYMEHSGNHSTHPVPSGKPPVLQSERLNLLYVPVAVAVAVVTAFIIICVHRCKKK
KTSAAEGPELVSLRTLDQHPVGTGDHRDATQLGFQPLMSAPGSTGSTEGA (SEQ ID
NO: 31).

### Example 5 Flow cytometry assay for binding of MICA antibody

Flow cytometry was used to detect the binding characteristics of the MICA antibody. The MICA antibody prepared in Example 1 (numbered as 5A1) and the control antibody mouse IgG1 (Biolegend, MG1-45) were added to the CHO-K1-MICA, CHO-K1-MICB, CHO-K1-Rhesus MIC1, and CHO-K1-Rhesus MICB cells obtained in Example 4, respectively. The signal strength after the antibody addition was used to determine the binding characteristics of the antibody and MICA, MICB, Rhesus MIC1, and Rhesus MICB proteins.

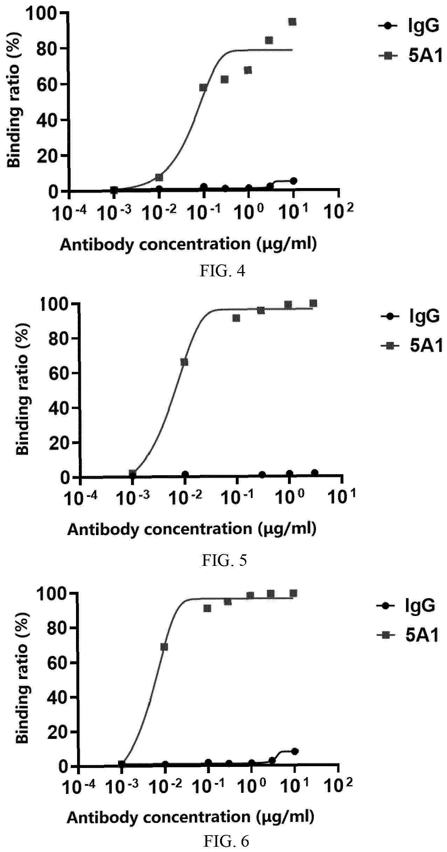

CHO-K1-MICA, CHO-K1-MICB, CHO-K1-Rhesus MIC1, and CHO-K1-Rhesus MICB cells were diluted to 2×10⁶/mL with PBS and added to 1.5 mL EP tube at 100 µL/tube. Goat serum at 10 µL/tube was added thereto and was blocked at 4°C for 30 min. MICA antibodies at different concentrations (10⁻⁴, 10⁻³, 10⁻², 10⁻¹, 10⁰, 10¹ µg/mL) were added and incubated at 4°C for 30min. The EP tube was added with 1 mL of PBS, and centrifuged at 3500 rpm at 4°C for 5 min. The supernatant was discarded and the precipitate was washed once more with PBS. After centrifugation, the supernatant was discarded. The cell-containing precipitate was resuspended with 100 µL/tube of PBS, 0.1 µL/tube of Alexa-647 labeled goat anti-mouse antibody secondary antibody (Biolegend, 405322) was added thereto and incubated at 4°C, under the condition of avoiding light, for 30 min. The cell-containing precipitate was washed twice with PBS and the supernatant was discarded after centrifugation. The cell-containing precipitate was resuspended with 200 µL/tube of PBS and detected by flow cytometry. The results are shown in FIGs. 3, 4, 5, and 6, further showing that the antibody of the present disclosure can bind to MICA, MICB, Rhesus MIC1, and Rhesus MICB proteins.

### Example 6 Flow cytometry assay for MICA antibody binding to tumor cell

Flow cytometry was used to detect the binding characteristics of MICA antibodies to tumor cells. MICA antibody (No. 5A1) and control mouse antibody IgG1 (Biolegend, MG1-45) were added to lung cancer NCI-H1299 cells, respectively. The signal strength after antibody addition was used to determine the binding characteristics of antibodies and tumor cells.

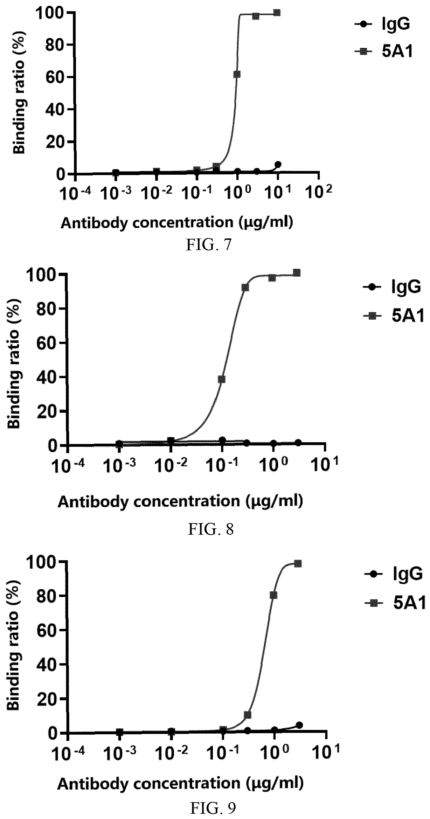

NCI-H1299 cells were diluted to 2 × 10⁶/mL with PBS and were added to a 1.5 mL EP tube at 100 µL/tube. Goat serum at 10 µL/tube was added thereto and was blocked at 4°C for 30 min. MICA antibodies, control antibody IgG1 at different concentrations (10⁻⁷, 10⁻⁶, 10⁻¹, 10⁻⁴, 10⁻³, 10⁻², 10⁻¹, 10⁰, 10¹, 10² µg/mL) were added and incubated at 4°C for 30 min. 1 mL of PBS was added to an EP tube. After 5 min of centrifugation at 4°C and 3500 rpm, the supernatant was discarded. The resulting precipitate was washed once more with PBS, and centrifuged once more. The supernatant was discarded. The cell-containing precipitate was resuspended with 100 µL/tube of PBS, 0.1 µL/tube Alexa-647 labeled goat anti-mouse antibody secondary antibody (Biolegend, 405322) was added thereto and incubated at 4°C, under the condition of avoiding light for 30 min. The cell-containing precipitate was washed twice with PBS and the supernatant was discarded after centrifugation. The cells were resuspended with 200 µL/tube of PBS, and detected by flow cytometry. The results are shown in FIG. 7, 8, and 9, further showing that the antibody of the present disclosure can bind to Melanoma A375 cells, lung cancer NCI-H1299 cells, and human erythroleukemia K562 cells.

### Example 7 Determination of antigenic epitope characteristics of MICA antibody

The Western blot assay was used to determine the antigenic epitope characteristics of the MICA antibody.

CHO-K1-MICA, CHO-K1 cells constructed in Example 4 were washed twice with PBS. The RIPA lysate was then added for lysis for 15 min on ice. After 5 min of centrifugation at 15,000 g at 4°C, the supernatant was collected. The lysate supernatant was subjected to protein quantification using a BCA kit. Sample was then prepared with SDS-PAGE loading buffer and the sample addition amount is 50 µg/well. After electrophoresis, the sample was transferred to a nitrocellulose membrane and blocked with 5% nonfat dried milk at room temperature for 1 h. MICA antibody diluted at 1: 2000 (w/v) was then added and incubated overnight at 4°C. The membrane was washed 5 min × 6 times with 1 ‰ PBST, then HRP-goat anti-mouse IgG diluted at 1: 5000 (v/v) was added and incubated at room temperature for 1 h. The membrane was washed 5 min × 6 times with 1‰ PBST. Chemiluminescence was detected using ECL substrate development. The results are shown in FIG.8, further showing that the antibody of the present disclosure can bind to a linear epitope of MICA protein.

### Example 8 Screening for the ability of the antibody to promote MICA binding to NKG2D

The MICA antibody can promote the signaling pathway of MICA and its receptor NKG2D by binding to the extracellular region of MICA. In this example, the effect of the MICA antibody on the binding of MICA to its receptor NKG2D was detected by flow cytometry.

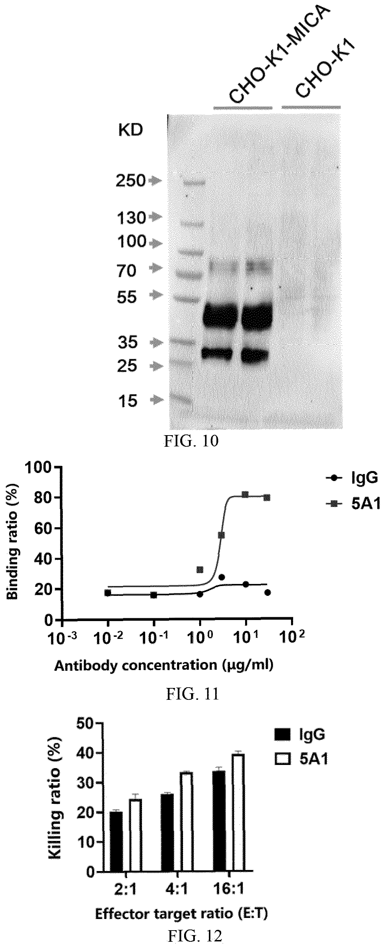

The CHO-K1-MICA cells obtained in Example 4 were diluted to 2 × 10⁶ cells/mL with PBS and were added to a 1.5 mL EP tube in a volume of 100 µL/tube. Mouse serum at 10 µL/tube was added thereto and was blocked at 4°C for 30 min. MICA antibodies No. 5A1 and the control antibody IgG1 (Biolegend, MG1-45) with a series of dilution gradients (10⁻⁴, 10⁻³, 10⁻², 10⁻¹, 10⁰, 10¹ µg/mL) were added and incubated at 4°C for 30 min. NKG2D extracellular region Fc fusion protein (NKG2D-Fc, SEQ ID NO: 32, obtained by expression in this laboratory) was added at 2 µg/tube and incubated for 30 min at 4°C. The EP tube was added with 1 mL of PBS and centrifuged at 3500 rpm at 4°C for 5 min. The supernatant was discarded and the precipitate was washed once more with PBS. After centrifugation, the supernatant was discarded. The cells were resuspended with 100 µL/tube of PBS, and 1 µL/tube of 647-labeled mouse anti-human antibody secondary antibody (Biolegend, HP6017) was added thereto, and incubated at 4°C, under the condition of avoiding light, for 30 min. The cell-containing precipitate was washed twice with PBS and the supernatant was discarded after centrifugation. The cells were resuspended with 200 µL/tube of PBS and detected by flow cytometry. The results are shown in FIG. 11. It can be seen that the antibody of the present disclosure can promote the binding of MICA to the receptor NKG2D.

IWSAVFLNSLFNQEVQIPLTESYCGPCPKNWICYKNNCYQFFDESKNWYES
QASCMSQNASLLKVYSKEDQDLLKLVKSYHWMGLVHIPTNGSWQWEDGSILSPNLL
TIIEMQKGDCALYASSFKGYIENCSTPNTYICMQRTVPKSCDKTHTCPPCPAPELLGGP
SVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK (SEQ ID NO: 32).

### Example 9 MICA antibody promotes anti-tumor effect of NK92 cells

This example is an in vitro killing assay to detect the effect of MICA antibody on killing tumor cells by NK92 cells (ATCC No. CRL-2407). A total of 2 groups were set up, in which the tumor cells used were melanoma A375 cells (ATCC No. CRL-1619). The specific experimental procedures were as follows.
(1) A375 tumor cells were washed twice with a serum-free DMEM medium, centrifuged at 4°C and 200 g for 5 min, and the supernatant was discarded. The resulting precipitate was resuspended with 1 mL of serum-free DMEM medium and CTV with a final concentration of 5 µM was added and incubated at 37°C for 30 min.
(2) After the incubation, 200 µL of pre-cooled fetal bovine serum was added to the culture system to stop the marker, and centrifuged at 4°C and 200 g for 5min, and the supernatant was discarded. The cell pellet was washed twice with a complete RPMI-1640 medium containing 10% fetal bovine serum, and the supernatant was discarded. The pellet was resuspended with the complete RPMI-1640 medium for cell counting and diluted to 1.25 × 10⁵ cells/mL with the complete RPMI-1640 medium.
(3) NK92 cells were diluted to a range of concentrations (2 × 10⁵/mL, 4 × 10⁵/mL, 8 × 10⁵/mL, 1.6 × 10⁶/mL) with the complete RPMI-1640 medium containing 10% fetal bovine serum.
(4) The diluted PBMC (NK92) was added to a 96-well round bottom plate in a volume of 100 µL/well.
(5) The MICA antibody (5A1) or control murine IgG was diluted to 50 µg/mL with the complete RPMI-1640 medium and the diluted MICA antibody or murine IgG was added to a 96-well round bottom plate in a volume of 20 µL/well.
(6) The diluted tumor cells were added to a 96-well round bottom plate in a volume of 80 µL/well.
(7) The 96-well round bottom plate in step (6) was centrifuged at 1500 rpm for 1 min at 4°C, and the centrifuged 96-well plate was incubated at 37°C for 4 h.
(8) 7-AAD (BD, 559925) was added to a 96-well round bottom plate in a volume of 1 µL/well and mixed well. All the liquid in the well was transferred into a flow cytometry tube and detected by using a flow cytometer. The specific experimental results are shown in FIG. 12, indicating that the antibody of the present disclosure can promote the killing of tumors by NK92 cells.

### Example 10: ELISA binding assay of MICA antibody

The binding characteristics of the MICA antibody and the α3 domains of MICA and MICB was detected by ELISA in this example. The obtained various MICA, MICB variant extracellular region α3 domains (MICAα3*-002, 004, 005, 008, MICBα3*-005) Fc fusion proteins (MICAα3-Fc, MICBα3-Fc) and control antibody IgG were coated into a 96-well plate by the inventors. The signal strength after antibody addition was used to determine the binding characteristics of the antibody to MICA and MICB.

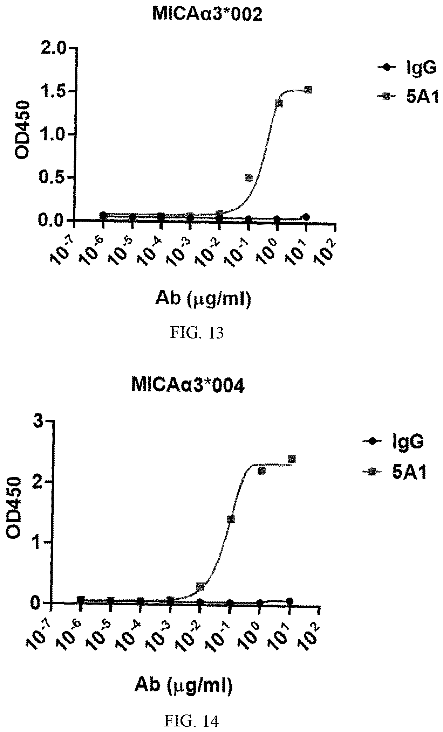

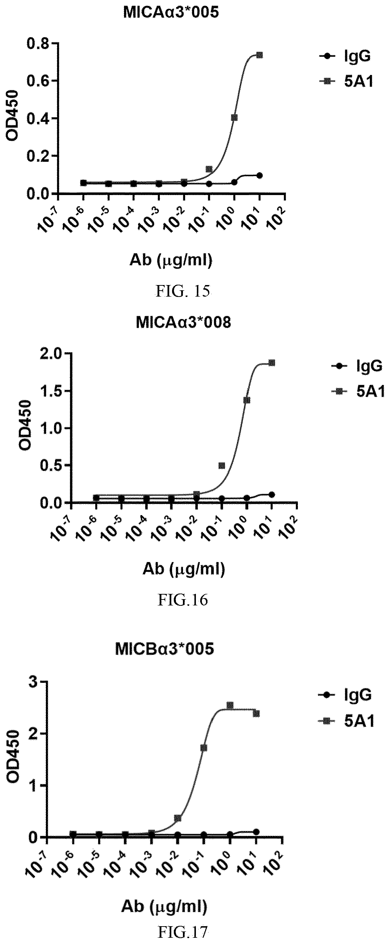

The fusion protein (MICA*002α3-Fc, amino acid sequence is as set forth in SEQ ID NO: 33; MICA*004α3-Fc, the amino acid sequence is as set forth in SEQ ID NO: 34; MICA*005α3-Fc, the amino acid sequence is as set forth in SEQ ID NO: 35; MICA*008α3-Fc, the amino acid sequence is as set forth in SEQ ID NO: 36; MICB*005α3-Fc, the amino acid sequence is as set forth in SEQ ID NO: 37) was diluted to 1 µg/ml with the PBS buffer and added to a 96-well plate in a volume of 100 µL/well and left overnight at 4°C. The PBS buffer solution was sucked off from the 96-well plate. The plate was washed 6 times with PBST (pH 7.2, PBS containing 0.1% Tween 20) buffer solution. 200 µL/well PBS/10% BSA was added and incubated at 37°C for 2 hours for blocking. The blocking solution was removed. The plate was washed 6 times with PBST. 100 µL/well MICA antibody to be tested diluted with PBST/0.05% BSA to an appropriate concentration (10⁻⁷, 10⁻⁶, 10⁻⁵, 10⁻⁴, 10⁻¹, 10⁻², 10⁻¹, 10⁰, 10¹, 10² µg/mL) were added, and incubated at 37°C for 1 h. The reaction system was removed. The plate was washed 6 times with PBST. HRP (horseradish peroxidase)-labeled anti-mouse antibody secondary antibody was diluted with PBST/0.05% BSA at 100 µL/well and incubated for 1 h at 37°C. The plate was washed 6 times with PBST, and 80 µL/well TMB (tetramethylbenzidine) was added. After 3 min of incubation at room temperature, and 80 µL/well 4M sulfuric acid was added to terminate the reaction. The absorbance at 450 mm was read with a microplate reader. The results are shown in FIGs. 13, 14, 15, 16, and 17, showing that the antibody of the present disclosure can bind to MICA*002, MICA*004, MICA*005, MICA*008, and MICB*005.

RTVPPMVNVTRSEASEGNITVTCRASGFYPWNITLSWRQDGVSLSHDTQQ
WGDVLPDGNGTYQTWVATRICQGEEQRFTCYMEHSGNHSTHPVPSGKVLVLQSHWP
KSCDKTHTCPPCPAPELLGGPSVFLFPPKPKDTLMISRTPEVTCVVVAVSHEDPEVKFN
WYVDGVEVHNAKTKPREEQYASTYRVVSVLTVLHQDWLNGKEYKCKVSNKALPAPI
EKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK
(SEQ ID NO: 33).

RRVPPMVNVTRSEASEGNITVTCRASSFYPRNITLTWRQDGVSLSHDTQQ
WGDVLPDGNGTYQTWVATRICQGEEQRFTCYMEHSGNHSTHPVPSGKVLVLQSHWP
KSCDKTHTCPPCPAPELLGGPSVFLFPPKPKDTLMISRTPEVTCVVVAVSHEDPEVKFN
WYVDGVEVHNAKTKPREEQYASTYRVVSVLTVLHQDWLNGKEYKCKVSNKALPAPI
EKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK
(SEQ ID NO: 34).

RTVPPMVNVTCSEVSEGNITVTCRASSFYPRNITLTWRQDGVSLSHNTQQW
GDVLPDGNGTYQTWVATRIRQGEEQRFTCYMEHSGNHGTHPVSGKVLVLQSHWPKS
CDKTHTCPPCPAPELLGGPSVFLFPPKPKDTLMISRTPEVTCVVVAVSHEDPEVKFNW
YVDGVEVHNAKTKPREEQYASTYRVVSVLTVLHQDWLNGKEYKCKVSNKALPAPIE
KTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
(SEQ ID NO: 35).

RTVPPMVNVTRSEASEGNITVTCRASSFYPRNIILTWRQDGVSLSHDTQQW
GGVLPDGNGTYQTWVATRICRGEEQRFTCYMEHSGNHSTHPVPSGKVLVLQSHWPK
SCDKTHTCPPCPAPELLGGPSVFLFPPKPKDTLMISRTPEVTCVVVAVSHEDPEVKFNW
YVDGVEVHNAKTKPREEQYASTYRVVSVLTVLHQDWLNGKEYKCKVSNKALPAPIE
KTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
(SEQ ID NO: 36).

RTVPPMVNVTCSEVSEGNITVTCRASSFYPRNITLTWRQDGVSLSHNTQQW
GDVLPDGNGTYQTWVATRIRQGEEQRFTCYMEHSGNHGTHPVPSGKVLALQSQWPK
SCDKTHTCPPCPAPELLGGPSVFLFPPKPKDTLMISRTPEVTCVVVAVSHEDPEVKFNW
YVDGVEVHNAKTKPREEQYASTYRVVSVLTVLHQDWLNGKEYKCKVSNKALPAPIE
KTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
(SEQ ID NO: 37).

In the description of this specification, the descriptions referring to the terms "one embodiment", "some embodiments", "example", "specific examples", or "some examples", etc. mean that a specific feature, structure, material, or characteristic described in connection with the embodiment or example is included in at least one embodiment or example of the present disclosure. In this specification, schematic representations of the terms above do not necessarily refer to the same embodiment or example. Furthermore, the specific features, structures, materials, or characteristics described may be combined in any suitable manner in any one or more embodiments or examples. Further, the different embodiments or examples and the features of the different embodiments or examples described in this specification can be integrated and combined by those skilled in the art without contradicting each other.

While embodiments of the present disclosure have been shown and described, it will be understood that the above-described embodiments are illustrative and should not be construed as limiting the present disclosure, and changes, modifications, substitutions, and alterations may be made to the above-mentioned embodiments by those skilled in the art without departing from the scope of the present disclosure.

## Claims

1. An antibody or antigen binding fragment, comprising CDR sequences selected from at least one of:
heavy-chain variable region CDR sequences: SEQ ID NOs: 1-3; and
light-chain variable region CDR sequences: SEQ ID NOs: 4-6.

2. The antibody or antigen binding fragment of claim 1, comprising:
a heavy chain CDR1 with an amino acid sequence as set forth in SEQ ID NO: 1 or a conservative modification form thereof,
a heavy chain CDR2 with an amino acid sequence as set forth in SEQ ID NO: 2 or a conservative modification form thereof,
a heavy chain CDR3 with an amino acid sequence as set forth in SEQ ID NO: 3 or a conservative modification form thereof,
a light chain CDR1 with an amino acid sequence as set forth in SEQ ID NO: 4 or a conservative modification form thereof,
a light chain CDR2 with an amino acid sequence as set forth in SEQ ID NO: 5 or a conservative modification form thereof, and
a light chain CDR3 with an amino acid sequence as set forth in SEQ ID NO: 6 or a conservative modification form thereof.

3. The antibody or antigen binding fragment of claim 1 or 2, comprising a heavy-chain variable region and a light-chain variable region, wherein:
(i) the heavy-chain variable region comprises an amino acid sequence having at least 80% homologous to at least one of an amino acid sequence as set forth in SEQ ID NO: 7 and a conservative modification form thereof; and/or, (ii) the light-chain variable region comprises an amino acid sequence having at least 80% homologous to at least one of an amino acid sequence as set forth in SEQ ID NO: 8 and a conservative modification form thereof.

4. The antibody or antigen binding fragment of any one of claims 1-3, wherein the heavy-chain variable region comprises an amino acid sequence having at least 90%, at least 95%, at least 96%, at least 97%, at least 98%, at least 99%, or 100% homologous to the heavy-chain variable region selected from (i); the light-chain variable region comprises an amino acid sequence having at least 90%, at least 95%, at least 96%, at least 97%, at least 98%, at least 99%, or 100% homologous to the light-chain variable region selected from (ii).

5. The antibody or antigen binding fragment of claim 3 or 4, wherein the heavy-chain variable region comprises an amino acid sequence as set forth in SEQ ID NO: 7 and the light-chain variable region comprises an amino acid sequence as set forth in SEQ ID NO: 8.

6. The antibody or antigen binding fragment of any one of claims 1-5, comprising: a heavy chain comprising an amino acid sequence as set forth in SEQ ID NO: 9 and a light chain comprising an amino acid sequence as set forth in SEQ ID NO: 10.

7. The antibody or antigen binding fragment thereof of any one of claims 1-6, wherein the antibody is a monoclonal antibody, a murine antibody, a chimeric antibody, a humanized antibody, a human antibody, Fv, a single chain antibody (scFv), Fab, Fab', Fab'-SH or F(ab')2.

8. The antibody or antigen binding fragment thereof of any one of claims 1-7, wherein the antibody or antigen binding fragment thereof is capable of binding to at least a portion of an amino acid sequence set forth in SEQ ID NO: 11.

9. An immunoconjugate, comprising a therapeutic agent and the antibody or antigen binding fragment of any one of claims 1-8, wherein the antibody or antigen binding fragment is conjugated to the therapeutic agent.

10. A composition, comprising the antibody or antigen binding fragment of any one of claims 1-8 and/or the immunoconjugate of claim 9.

11. A kit for detecting MICA and/or MICB, comprising the antibody or antigen binding fragment of any one of claims 1-8.

12. Use of the antibody or antigen binding fragment thereof of any one of claims 1-8 in preparing a kit configured to detect MICA and/or MICB.

13. A medicament comprising the antibody or antigen binding fragment of any one of claims 1-8 and/or the immunoconjugate of claim 9 and/or the composition of claim 10.

14. Use of the antibody or antigen binding fragment of any one of claims 1-8 and/or the immunoconjugate of claim 9 and/or the composition of claim 10 in preparing a medicament for prevention and/or treatment of a MICA and/or MICB-mediated disease;
optionally, the MICA and/or MICB-mediated disease is a cancer, a transplant rejection, an autoimmune disease, or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

15. A nucleic acid comprising a sequence encoding the antibody or antigen binding fragment of any one of claims 1-8,
optionally, the nucleic acid has a nucleotide sequence as set forth in SEQ ID NO: 12 or 13.

16. A recombinant vector or transformant comprising the nucleic acid of claim 15.

17. A recombinant cell carrying the nucleic acid of claim 15, the expression vector or transformant of claim 16, or expressing the antibody or antigen binding fragment of any one of claims 1-8.

18. Use of the antibody or antigen binding fragment of any one of claims 1-8, the immunoconjugate of claim 9, the composition of claim 10, the medicament of claim 13, the nucleic acid of claim 15, the recombinant vector or transformant of claim 16, or the recombinant cell of claim 17 in treating and/or preventing of a MICA and/or MICB-mediated disease.

19. The use of claim 18, wherein the MICA and/or MICB-mediated disease is a cancer, a transplant rejection, an autoimmune disease, or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

20. A method for treating and/or preventing a MICA and/or MICB-mediated disease comprising administering to a subject at least one of:
1) the antibody or antigen binding fragment thereof of any one of claims 1-8;
2) the immunoconjugate of claim 9;
3) the composition of claim 10;
4) the medicament of claim 13;
5) the nucleic acid of claim 15;
6) the recombinant vector or transformant of claim 16; and
7) the recombinant cell of claim 17.

21. The method of claim 20, wherein the MICA and/or MICB-mediated disease is a cancer, a transplant rejection, an autoimmune disease, or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

22. A method for diagnosing whether a subject has a MICA and/or MICB-mediated disease, comprising detecting MICA and/or MICB in a sample to be tested using at least one of:
1) the antibody or antigen binding fragment thereof of any one of claims 1-8;
2) the nucleic acid of claim 15;
3) the recombinant vector or transformant of claim 16; and
4) the recombinant cell of claim 17; and
determining the content of MICA and/or MICB in the sample to be tested based on the detection result of the MICA and/or MICB.

23. The method of claim 22, wherein the content of MICA and/or MICB in the sample to be tested that is not lower than the minimum standard for the disease is an indication that the sample to be tested is derived from a patient having a MICA and/or MICB-mediated disease.

24. The method of claim 22, wherein the MICA and/or MICB-mediated disease is a cancer, a transplant rejection, an autoimmune disease, or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

25. Use of at least one of the following in diagnosing whether a subject has a MICA and/or MICB-mediated disease:
1) the antibody or antigen binding fragment thereof of any one of claims 1-8;
2) the nucleic acid of claim 15;
3) the recombinant vector or transformant of claim 16; and
4) the recombinant cell of claim 17.

26. The use of claim 25, wherein the MICA and/or MICB-mediated disease is a cancer, a transplant rejection, an autoimmune disease, or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.
